# EUROPEAN PATENT APPLICATION

(11) **EP 2 546 219 A1**
(43) Date of publication of application: **16.01.2013**
(21) Application number: 11753377.8
(22) Date of filing: 08.03.2011
(51) Int. Cl.: C07C 1/20, C07C 15/14, C07B 61/00

(54) **CARBON NANORING AND METHOD FOR PRODUCING A RING-SHAPED COMPOUND SUITABLE AS A STARTING MATERIAL FOR PRODUCTION OF THE SAME**

(30) Priority: 01.09.2010 JP 2010196175; 08.03.2010 JP 2010051045
(71) Applicant: National University Corporation Nagoya University, Aichi 464-8601 (JP)
(72) Inventor: ITAMI, Kenichiro, Nagoya-shi Aichi 464-8601 (JP); SEGAWA, Yasutomo, Nagoya-shi Aichi 464-8601 (JP); MIYAMOTO, Shinpei, Nagoya-shi Aichi 464-8601 (JP); OMACHI, Haruka, Nagoya-shi Aichi 464-8601 (JP); MATSUURA, Sanae, Nagoya-shi Aichi 464-8601 (JP); SENEL, Petr, Nagoya-shi Aichi 464-8601 (JP)
(74) Representative: Vossius & Partner
(86) International application number: PCT/JP2011/055423
(87) International publication number: WO 2011/111719

(57) **Abstract**

The present invention produces Cyclic Compound (1) in which organic ring groups including cyclohexane rings and benzene rings are continuously bonded, using a compound having at least one cyclohexane ring and benzene rings with halogen atoms at the two terminuses, in the presence of a nickel compound (bis(1,5-cyclooctadiene)nickel, etc.). Thereafter, by converting the cyclohexane rings in Cyclic Compound (1) into benzene rings, a desired carbon nanoring can be obtained. Thereby, the present invention efficiently produces a carbon nanoring made of a compound having a cyclic structure in which a desired number of organic ring groups are continuously bonded, with a short production process.

## Description

### Technical Field

The present invention relates to a method for producing a carbon nanoring in which organic ring groups such as bivalent aromatic hydrocarbon groups are circularly bonded, and a method for producing a cyclic compound suitable as a starting material for the carbon nanoring.

In the present specification, "organic ring groups" composing a carbon nanoring designate bivalent aromatic hydrocarbons, such as a phenylene group or a naphthylene group; bivalent alicyclic hydrocarbon groups, such as a cyclohexylene group; bivalent heterocyclic groups, such as a pyridylidene group or a pyrimidinylidene group; or derivative groups thereof in which the hydrogen atoms bonded to the carbon atoms of these groups are substituted with functional groups.

### Background Art

Hitherto-known nano structures containing carbon atoms include carbon nanotubes made of a cylindrically-rolled two-dimensional graphene sheet, and cyclic carbon nanotubes containing such carbon nanotubes.

Carbon nanotubes have extremely high mechanical strength and high temperature resistance, and efficiently discharge electrons under voltage application. With these advantageous properties, carbon nanotubes are expected to be applied to various fields, including chemistry, electronics, and life sciences.

Known methods of manufacturing carbon nanotubes include arc discharge, laser furnaces, chemical vapor deposition, and the like. However, these methods have a disadvantage in that they can only produce mixtures of carbon nanotubes with various diameters and lengths.

As a replacement for tubular nano structures such as carbon nanotubes having a certain length derived from a continuous linkage of carbon atoms, recent studies have focused attention on cyclic nano structures. For example, Non-Patent Literature 1 discloses a method for producing a cycloparaphenylene compound obtained by using cyclohexanedione and diiodobenzene. This compound has 12 continuously bonded phenylene groups, which are bivalent aromatic hydrocarbon groups.

### Citation List

■ Non-Patent Literature

### Non-patent literature 1

Takaba, H.; Omachi, H.; Yamamoto, Y.; Bouffard, J.; Itami, K. Angew. Chem. Int. Ed.2009, 48, 6112

### Summary of Invention

### Technical Problem

Non-Patent Literature 1 discloses a cycloparaphenylene compound having 12 continuously bonded phenylene groups, which is produced in the presence of a palladium compound; and a production method thereof. In the method of Non-Patent Literature 1 for producing a cycloparaphenylene compound having 12 continuously bonded phenylene groups, starting compounds are individually coupled together into a cyclic tetramer, thereby synthesizing a cycloparaphenylene compound having 12 continuously bonded phenylene groups. However, this method requires a separate reaction step for each of the starting compounds. For example, in the synthesis of a cycloparaphenylene compound having 12 continuously bonded phenylene groups, it is necessary to perform 4 steps to obtain the cyclic tetramer of the starting compounds. Such a complicated and cumbersome production method is very costly. Thus, this method is insufficient for the efficient production of carbon nanorings.

An object of the present invention is to provide a method for efficiently producing a carbon nanoring made of a cyclic compound having a desired number of continuously bonded organic ring groups, with a short production process; a carbon nanoring produced by the method; and a carbon nanoring made of a cycloparaphenylene compound.

### Solution to Problem

In light of the above object, the inventors of the present invention conducted intensive research, and found that a carbon nanoring in which a desired number of organic ring groups are circularly bonded can be obtained with a short production process by the following scheme represented by Reaction Formula 1.

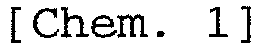

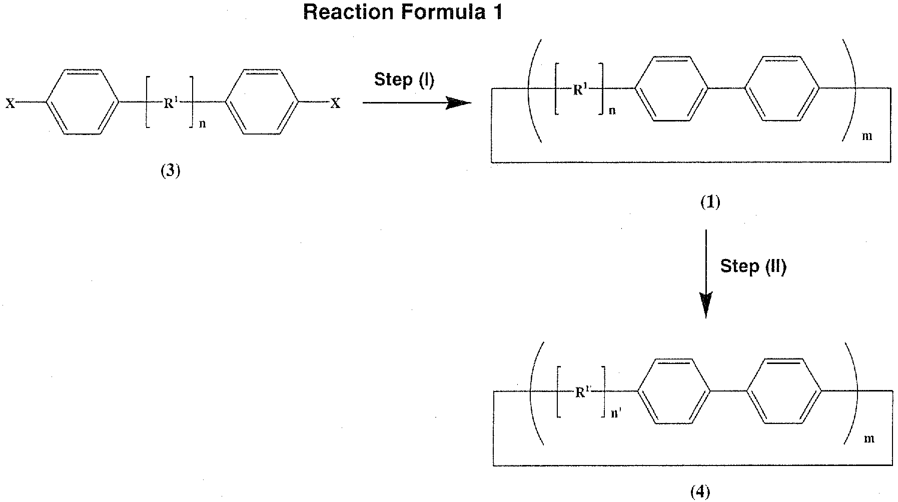

wherein n R¹ is the same or different, and each represents a bivalent aromatic hydrocarbon group, a bivalent alicyclic hydrocarbon group, a bivalent heterocyclic group, or a derivative group thereof; at least one R¹ is a bivalent group represented by Formula (2):

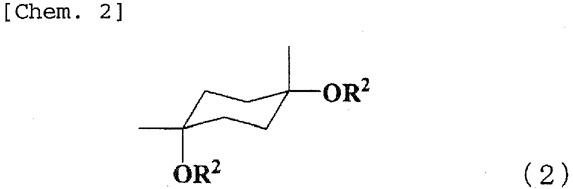

wherein R² is the same or different, and each represents a hydrogen atom or a protecting group for a hydroxy group;
n' R^{1'} is the same or different, and each represents a bivalent aromatic hydrocarbon group, a bivalent alicyclic hydrocarbon group, a bivalent heterocyclic group, or a derivative group thereof; at least one R^{1'} is a p-phenylene group; n is an integer of 1 or more; n' is an integer of 1 or more; and m is an integer of 2 or more.

The present invention was completed as a result of further research based on the above finding. Specifically, the present invention encompasses the following production methods for cyclic compounds and carbon nanorings; cyclic compounds; and production methods thereof as defined in Items 1 to 13.

### Item 1.

A method for producing Cyclic Compound (1) represented by General Formula (1): 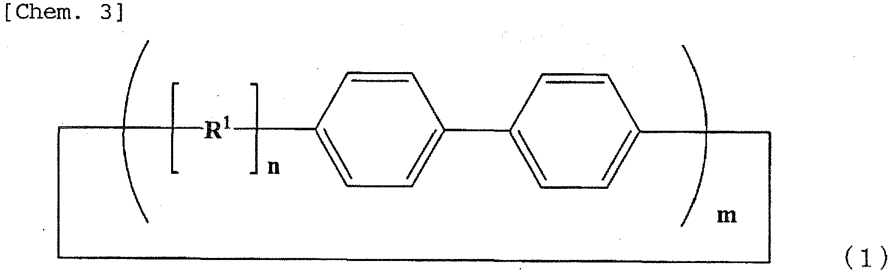 wherein n R¹ is the same or different, and each represents a bivalent aromatic hydrocarbon group, a bivalent alicyclic hydrocarbon group, a bivalent heterocyclic group, or a derivative group thereof (hereinafter, they may be simply referred to as "organic ring groups"); at least one R¹ is a bivalent group (2) represented by Formula (2): 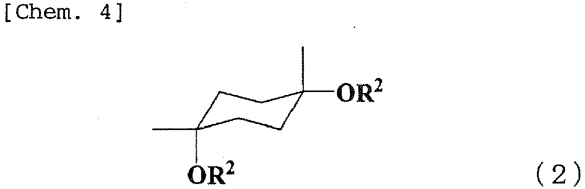 wherein R² is the same or different, and each represents a hydrogen atom or a protecting group for a hydroxy group;
n is an integer of 1 or more; and m is an integer of 2 or more,
the method comprising the step of:
(I) forming a cyclic compound represented by General Formula (1) in the presence of a nickel compound using Compound (3) represented by General Formula (3): 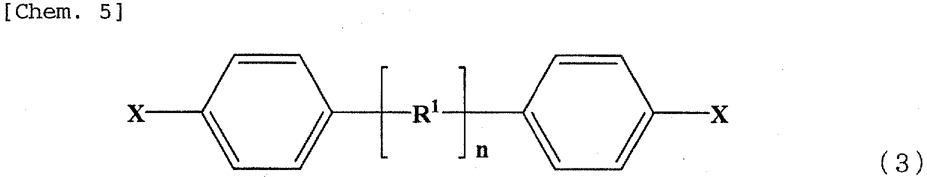 wherein R¹ and n are as defined above; X is the same or different, and each represents a halogen atom.

### Item 2.

The method for producing the cyclic compound according to Item 1, wherein the compound represented by General Formula (3) is Compound (3a) represented by General Formula (3a): 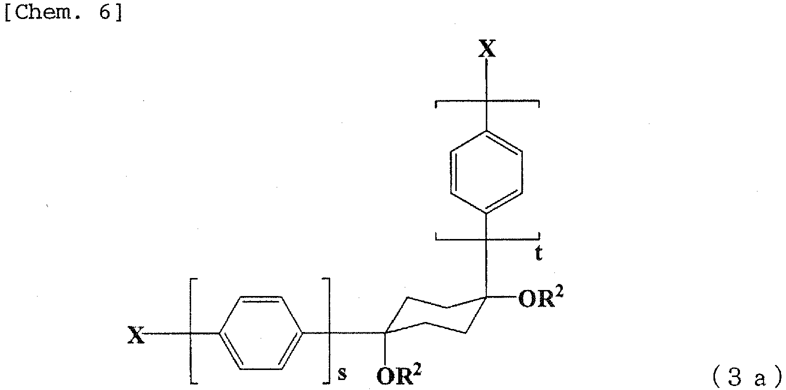 wherein X and R² are as defined above; s is an integer of 1 or more; t is an integer of 1 or more; and s+t = n+1 (n is as defined above).

### Item 3.

The method for producing the cyclic compound according to Item 1, wherein the compound represented by General Formula (3) is Compound (3c) represented by General Formula (3b): 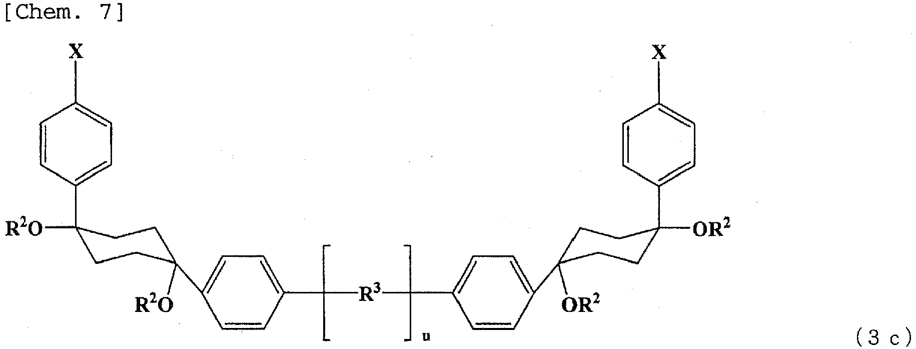 wherein X and R² are as defined above; u R³ is the same or different, and each represents a bivalent aromatic hydrocarbon group, a bivalent alicyclic hydrocarbon group, a bivalent heterocyclic group, or a derivative group thereof; u is an integer of 1 or more; and u = n-4.

### Item 4.

The method for producing the cyclic compound according to Item 1 or 2,
wherein:
the compound represented by General Formula (3) is Compound (3a-1) represented by General Formula (3a-1): 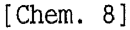 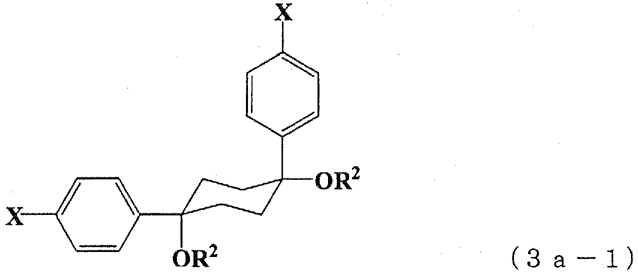
   wherein X and R² are as defined above,
   and wherein m in General Formula (1) is 4.

### Item 5.

The method for producing the cyclic compound according to Item 1 or 2,
wherein:
the compound represented by General Formula (3) is Compound (3a-1) represented by General Formula (3a-1): 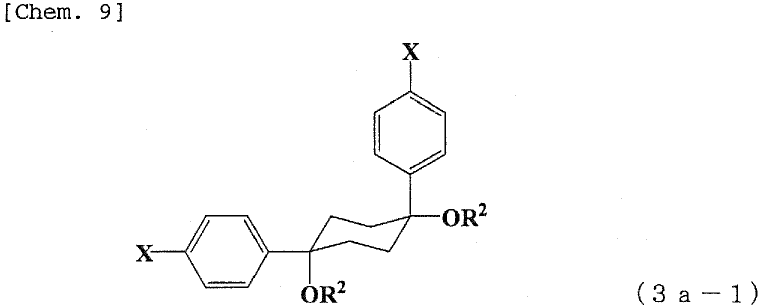
   wherein X and R² are as defined above,
   and wherein m in General Formula (1) is 3.

### Item 6.

The method for producing the cyclic compound according to Item 3, wherein m in General Formula (1) is 2, and u in General Formula (3b) is 1 or 2.

### Item 7.

A method for producing Carbon Nanoring (4) represented by General Formula (4): 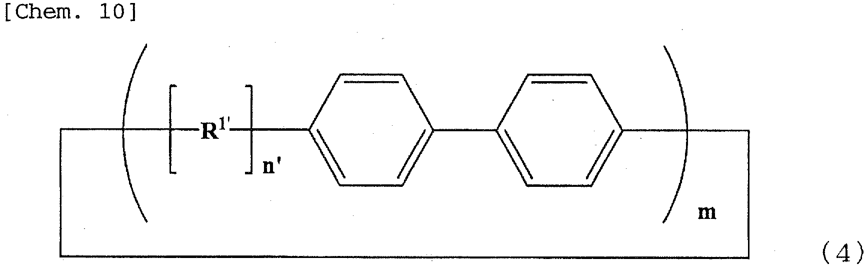 wherein n' R^{1'} is the same or different, and each represents a bivalent aromatic hydrocarbon group, a bivalent alicyclic hydrocarbon group, a bivalent heterocyclic group, or a derivative group thereof; at least one R^{1'} is a p-phenylene group; n' is an integer of 1 or more; and m is an integer of 2 or more,
the method comprising the step of:
(II) converting cyclohexane rings of Cyclic Compound (1) obtained by the method according to any one of Items 1 to 6 into benzene rings.

### Item 8.

The method for producing the carbon nanoring according to Item 7, wherein Step (II) performs an oxidation reaction of Cyclic Compound (1).

### Item 9.

The method for producing the carbon nanoring according to Item 7 or 8,
wherein:
R^{1'} in General Formula (4) is represented by General Formula (2), and wherein n' is 1 and m is 4.

### Item 10.

The method for producing the carbon nanoring according to Item 7 or 8,
wherein:
R^{1'} in General Formula (4) is represented by General Formula (2), and wherein n' is 1 and m is 3.

### Item 11.

The method for producing the carbon nanoring according to Item 7 or 8,
wherein:
R^{1'} in General Formula (4) is represented by General Formula (5): 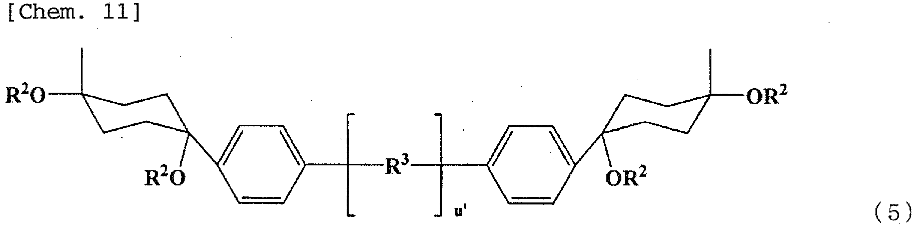
   wherein R² and R³ are as defined above; u' is 1 or 2,
   and wherein n' is 1 and m is 2.

### Item 12.

A cyclic compound represented by General Formula (1b): 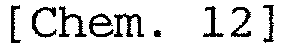 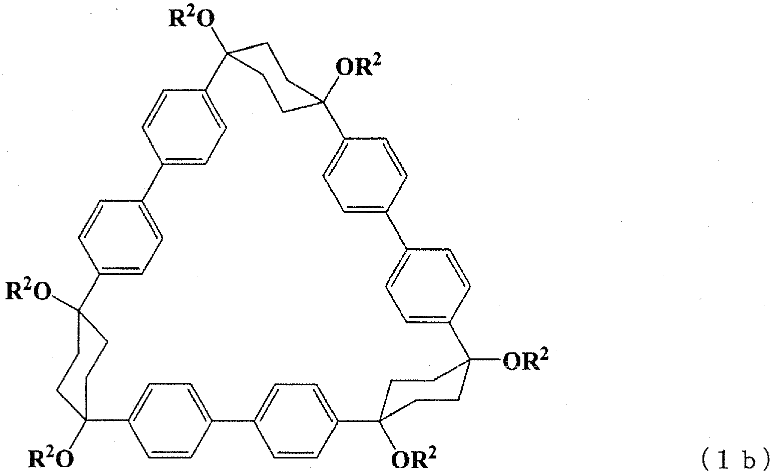 wherein R² is the same or different, and each represents a hydrogen atom or a protecting group for a hydroxy group.

### Item 13.

A method for producing Carbon Nanoring (4b) comprising 9 p-phenylene groups, represented by Formula (4b): 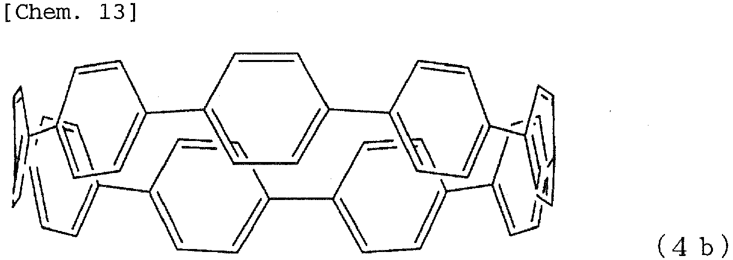 the method comprising the step of:
(IIb) converting cyclohexane rings of a cyclic compound represented by General Formula (1b): 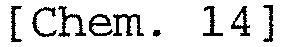 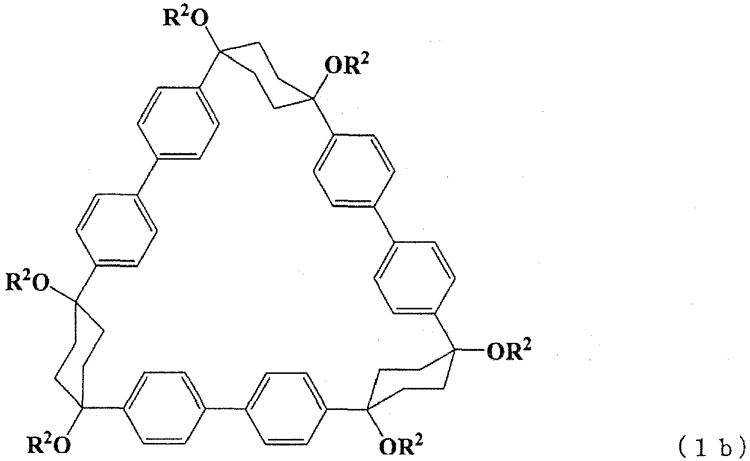 wherein R² is the same or different, and each represents a hydrogen atom or a protecting group for a hydroxy group,
   into benzene rings.

### Advantageous Effects of Invention

Compound (3) used as a starting material for the present invention contains, at each molecular terminus, a benzene ring having a halogen atom, and the aforementioned bivalent group (2), i.e., a group derived from a cyclohexane ring. The cyclohexane ring is attached to a benzene ring, an organic ring group, or the like, at the 1-position and 4-position, forming a nonlinear (L-shaped) structure of chair conformation in which the groups attached to the cyclohexane ring, such as a benzene ring, an organic ring group, etc., are at axial and equatorial positions. Compound (3) used as a starting material is not limited to those having only one cyclohexane ring, and may have two or more cyclohexane rings. Compound (3) generally has an overall U-shape.

By subjecting Compound (3) to a homocoupling reaction in the presence of a nickel compound, Cyclic Compound (1) in which an arbitrary number of organic ring groups are continuously bonded is formed. Further, by converting the cyclohexane rings into benzene rings, a carbon nanoring can be efficiently obtained with a short production process, specifically, with two steps. This reduces production costs.

For example, by using Compound (3a) as Compound (3), it is possible to accurately design and obtain a carbon nanoring made of a cyclic compound, which is composed of carbon atoms and hydrogen atoms, and is structured such that an arbitrary number of organic ring groups (phenylene groups) are continuously bonded.

More specifically, by using Compound (3a-1) having an overall L-shape as Compound (3), the resulting compound obtained as Cyclic Compound (1) is a tetramer (the compound represented by General Formula (1a-1)) of Compound (3a-1). Thereafter, by converting the cyclohexane rings into benzene rings, a carbon nanoring made of a cycloparaphenylene compound having 12 continuously bonded phenylene groups can be efficiently obtained with a short production process. Further, in this case, it is possible to obtain, as Cyclic Compound (1), not only a tetramer but also Cyclic Compound (1b) which is a trimer of Compound (3a-1). Thereafter, by converting the cyclohexane rings into benzene rings, a carbon nanoring made of a cycloparaphenylene compound in which 9 phenylene groups are continuously bonded can be efficiently obtained with a short production process.

Further, by using Compound (3c) having an overall U-shape as Compound (3), it is possible to obtain a dimer (the compound represented by General Formula (1c)) of Compound (3c) as Cyclic Compound (1). Thereafter, by converting the cyclohexane rings into benzene rings, a carbon nanoring made of a cycloparaphenylene compound in which 14, 16, etc., phenylene groups are continuously bonded can be efficiently obtained with a short production process.

As described above, the present invention enables efficient production of an accurately designed carbon nanoring in which an arbitrary number of organic ring groups are continuously bonded, with a short production process. The present invention is also useful for the synthesis of a carbon nanotube having a diameter corresponding to the number of organic ring groups, such as phenylene groups.

The carbon nanoring produced by the method of the present invention is particularly suitable as an electronic industry material, luminescence material, and the like.

### Brief Description Of Drawings

[Fig. 1]
   A drawing made by the Oak Ridge Thermal-Ellipsoid Plot (ORTEP) program, showing a structure of a [12]cycloparaphenylene crystal containing n-hexane molecules.
[Fig. 2]
   A drawing made by the Oak Ridge Thermal-Ellipsoid Plot (ORTEP) program, showing part of an assembly of [12]cycloparaphenylene crystals.
[Fig. 3]
   A drawing made by the Oak Ridge Thermal-Ellipsoid Plot (ORTEP) program, showing a crystalline Cyclic Compound (1b-1) containing ethyl acetate.
[Fig. 4]
   A drawing made by the Oak Ridge Thermal-Ellipsoid Plot (ORTEP) program, showing the structure of a [9]-cycloparaphenylene crystal containing THF.

### Description of Embodiments

In the present invention, Carbon Nanoring (4) can be produced through the following scheme represented by Reaction Formula 1: 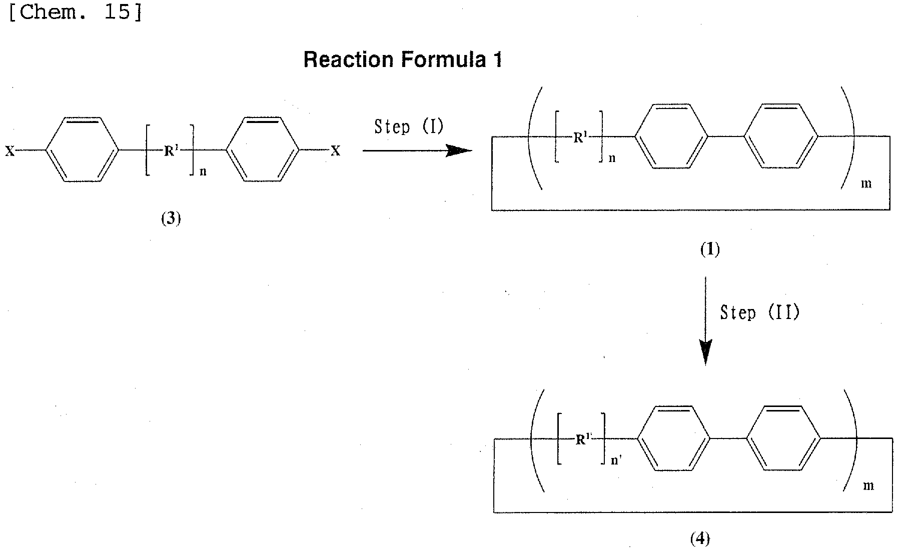 wherein n R¹ is the same or different, and each represents a bivalent aromatic hydrocarbon group, a bivalent alicyclic hydrocarbon group, a bivalent heterocyclic group, or a derivative group thereof; at least one R¹ is a bivalent group represented by Formula (2): 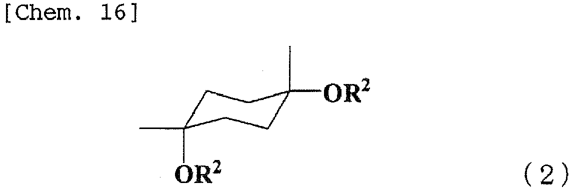
wherein R² is the same or different, and each represents a hydrogen atom or a protecting group for a hydroxy group;
n' R^{1'} is the same or different, and each represents a bivalent aromatic hydrocarbon group, a bivalent alicyclic hydrocarbon group, a bivalent heterocyclic group, or a derivative group thereof; at least one R^{1'} is a p-phenylene group; n is an integer of 1 or more; n' is an integer of 1 or more; and m is an integer of 2 or more.

In General Formula (3) in Reaction Formula 1, X is not particularly limited insofar as it is a halogen atom. Examples of X include a fluorine atom, a chlorine atom, a bromine atom and an iodine atom. In the present invention, a bromine atom and an iodine atom are preferable to ensure a smooth reaction in Step (I), and an iodine atom is more preferable. In General Formula (3), the two Xs may be the same or different.

In General Formulae (1) and (3) in Reaction Formula 1, R¹ represents one or more kinds of organic ring groups (hereinafter, they may be simply referred to as "organic ring groups") selected from bivalent aromatic hydrocarbon groups, bivalent alicyclic hydrocarbon groups, bivalent heterocyclic groups and derivative groups thereof. At least one R¹ is a cyclohexylene derivative group represented by Formula (2).

In other words, as long as one of R¹ is a cyclohexylene derivative group represented by General Formula (2), another R¹ may be a group other than a cyclohexylene derivative group.

In General Formula (2), R² is a hydrogen atom or a protecting group for a hydroxy group. The protecting group for a hydroxy group is not particularly limited. Examples thereof include an alkyl group, an acyl group, a silyl group, an alkoxyalkyl group, a tetrahydropyranyl group (THP), and a benzyl group. An alkoxyalkyl group is preferable.

The alkyl group may have a branched, linear, or cyclic structure. The carbon number of the alkyl group is not particularly limited; however, the carbon number is preferably 1 to 20, more preferably 1 to 10, and still more preferably 1 to 5. Specifically, methyl, ethyl, n-propyl, isopropyl, n-butyl, isobutyl, tert-butyl, sec-butyl, pentyl, cyclohexyl, or the like may be used as the alkyl group.

The acyl group designates a monovalent group represented by -CO-R^{2a}. R^{2a} of the acyl group is an alkyl group, and may have a branched or linear structure. The carbon number of the alkyl group of the acyl group is not particularly limited; however, the carbon number is preferably 1 to 20, more preferably 1 to 10, and still more preferably 1 to 5. Specific examples thereof include acetyl, propionyl, and the like.

The silyl group designates a monovalent group represented by -SiR^{1b}3 (a plurality of R^{1b} groups may be the same or different). Examples of R^{1b} of the silyl group include a hydrogen atom, a hydroxy group, an alkyl group, an alkoxy group, an allyl group, an aryl group, and an amino group. When R^{1b} is an alkyl group or an alkoxy group, the alkyl group of the alkyl or alkoxy group may have a branched or linear structure. The carbon number of the alkyl group is not particularly limited; however, the carbon number is preferably 1 to 20, more preferably 1 to 10, and still more preferably 1 to 5. Further, when R^{1b} is an aryl group, the number of aromatic hydrocarbon rings of the aryl group is not particularly limited; however, the number of aromatic hydrocarbon rings is preferably 1 to 3. If the aryl group has a plurality of aromatic hydrocarbon rings, the rings may be condensed. The aryl group may have one or more other substituents (functional groups). For example, each aromatic ring of the aryl group may have one or more other substituents (functional groups). The bonding sites of these substituents (functional groups) may be either the ortho-position, meth-position, or para-position. Specifically, examples of substituents (functional groups) include one or more of: a halogen atom, an alkyl group, an alkenyl group, a nitro group, an amino group, a hydroxy group, and an alkoxy group. When these substituents (functional groups) are bonded with a carbon atom of the aromatic ring, the bonding sites of the substituents (functional groups) may be either the ortho-position, meth-position, or para-position. Specifically, examples of substituents include a trimethylsilyl group, a triethylsilyl group, and a t-butyldimethylsilyl group.

The alkoxyalkyl group designates a monovalent group represented by -R^{1c}-O-R^{1d}. R^{1c} of the alkoxyalkyl group is an alkylene group having a linear or branched structure. The carbon number thereof is preferably 1 to 20, more preferably 1 to 10, and still more preferably 1 to 5. Further, R^{1d} is an alkyl group having a linear or branched structure. The carbon number thereof is preferably 1 to 20, more preferably 1 to 10, and still more preferably 1 to 5. A preferable example of the alkoxyalkyl group is a methoxymethyl group (-CH₂-O-CH₃, may also be referred to as "-MOM" hereinafter).

The protecting group (in particular, a methoxymethyl group) is substituted with a hydrogen atom of alcohol (hydroxy group), thereby serving as a protecting group of the alcohol.

Further, among the protecting groups, methoxymethyl is obtained by reacting the alcohol to be protected with chloromethylmethylether (Cl-CH₂-O-CH₃).

Further, in General Formula (2), the two R¹ groups are the same or different. When the later-described Compound (1) is used as a starting material for Carbon Nanoring (4), R¹ is preferably a methoxymethyl group (-CH₂-O-CH₃).

The organic ring group other than the cyclohexylene derivative group is not limited insofar as it is as defined above. Specifically, examples thereof include bivalent groups containing organic rings selected from aromatic rings, cycloalkanes and heterocyclic rings, obtained by detaching a hydrogen atom from each of the two carbon atoms of the organic ring. The hydrogen atom bonded to each of the two carbon atoms of the organic ring may be a derivative group (bivalent derivative group) substituted with a functional group. When there are a plurality of organic ring groups other than a cyclohexylene derivative groups, they may be the same or different.

In addition to benzene rings, examples of the aromatic rings also include rings resulting from the condensation of multiple benzene rings (benzene condensed rings) and rings resulting from the condensation of benzene and other rings (hereinafter, these rings resulting from the condensation of multiple benzene rings and rings resulting from the condensation of benzene and other rings may be collectively referred to as "condensed rings"). Examples of the condensed rings include a pentalene ring, indene ring, naphthalene ring, anthracene ring, tetracene ring, pentacene ring, pyrene ring, perylene ring, triphenylene ring, azulene ring, heptalene ring, biphenylene ring, indacene ring, acenaphthylene ring, fluorene ring, phenalene ring, and phenanthrene ring.

The cycloalkanes are not limited insofar as they have 3 to 10 carbon atoms; Examples include a cyclopropane ring, cyclohexane ring, and the like. The cyclohexane ring has a nonplanar conformation. The cyclohexane ring(s) used as organic ring group(s) other than cyclohexylene derivative groups may have a boat or a twist-boat conformation (structure), excluding a chair conformation (L-shaped structure).

Examples of the heterocyclic rings include heterocyclic rings having at least one atom selected from a nitrogen atom, oxygen atom, boron atom, phosphorus atom, silicon atom and sulfur atom (namely, heterocyclic aromatic rings and heterocyclic aliphatic rings, in particular, heterocyclic aromatic rings). Examples of heterocyclic rings include a furan ring, thiophene ring, pyrrole ring, silole ring, borole ring, phosphole ring, oxazole ring, thiazole ring, pyridine ring, pyridazine ring, pyrimidine ring, and pyrazine ring. Heterocyclic condensed rings (thienothiophene rings, quinoline rings) obtained by combining these rings, these rings and benzene rings, or these rings and the aforementioned condensed rings, may also be used.

Further, when the another organic ring group of R¹ is a cycloalkane (excluding cyclohexanes having a chair conformation), the presence/absence of substituents (functional groups) is not particularly limited; however, a bivalent derivative group resulting from substitution of a hydrogen atom bonded to a carbon atom of the alicyclic hydrocarbon group with another functional group is preferable. In this case, a preferable substituent (functional group) is -OR² in General Formula (2). When one or more R¹ are cycloalkylene groups having -OR² (excluding a cyclohexylene derivative group represented by General Formula (2)), the carbon-carbon bonds forming the ring may be converted into double bonds in Step (II) (described later).

In the present invention, the one or more other organic ring groups of R¹ are preferably, among the aforementioned rings, a group containing a bivalent 6-membered aromatic ring or a bivalent 6-membered heterocyclic aromatic ring, with a bonding site at the para-positions.

Further, as the other organic ring groups of R¹, a group derived from a monocyclic or a condensed ring is preferable. A group derived from a monocyclic ring is more preferable. More specifically, the one or more other organic ring groups of R¹ are preferably a phenylene group (in particular, a p-phenylene group) and a naphthylene group (in particular, a 1,5-naphthylene group or 2,6-naphthylene group). A phenylene group (in particular, a p-phenylene group) is more preferable.

R^{1'} is the same as R¹ except that the bivalent group derived from the cyclohexane ring represented in General Formula (2) is converted into a p-phenylene group. Therefore, R^{1'} is not particularly limited insofar as it is the same as the one or more other organic ring groups of R¹. More specifically, examples thereof include bivalent groups having organic rings selected from aromatic rings, cycloalkanes and heterocyclic rings, and result from elimination of a hydrogen atom from each of the two carbon atoms of the organic ring. The hydrogen atom attached to each of the two carbon atoms of the organic ring may be a derivative group (bivalent derivative group) substituted with a functional group. When there are a plurality of organic ring groups other than a cyclohexylene derivative groups, they may be the same or different.

In addition to benzene rings, examples of the aromatic rings also include condensed rings. Examples of the condensed rings include a pentalene ring, indene ring, naphthalene ring, anthracene ring, tetracene ring, pentacene ring, pyrene ring, perylene ring, triphenylene ring, azulene ring, heptalene ring, biphenylene ring, indacene ring, acenaphthylene ring, fluorene ring, phenalene ring, and phenanthrene ring. However, since at least one R¹ is a bivalent group derived from the cyclohexane ring represented by General Formula (2), at least one R^{1'} is also a benzene ring.

The cycloalkanes are not limited insofar as they have 3 to 10 carbon atoms; Examples include a cyclopropane ring, cyclohexane ring, and the like. The cyclohexane ring has a nonplanar conformation. Unlike the other organic ring groups of R¹, the cyclohexane ring(s) used as one or more other organic ring groups of R^{1'} may have a chair conformation (L-shaped structure), a boat conformation, or a twist-boat conformation.

Examples of the heterocyclic rings include heterocyclic rings having at least one atom selected from a nitrogen atom, oxygen atom, boron atom, phosphorus atom, silicon atom and sulfur atom (namely, heterocyclic aromatic rings and heterocyclic aliphatic rings, in particular, heterocyclic aromatic rings). Examples of heterocyclic rings include a furan ring, thiophene ring, pyrrole ring, silole ring, borole ring, phosphole ring, oxazole ring, thiazole ring, pyridine ring, pyridazine ring, pyrimidine ring, and pyrazine ring. Heterocyclic condensed rings (thienothiophene rings, quinoline rings) obtained by combining these rings, these rings and benzene rings, or these rings and the aforementioned condensed rings, may also be used.

Further, when R^{1'} is an organic ring group of a cycloalkane, the presence/absence of substituents (functional groups) is not particularly limited; however, a bivalent derivative group resulting from substitution of a hydrogen atom bonded to a carbon atom of the alicyclic hydrocarbon group with another functional group is preferable. In this case, a preferable substituent (functional group) is -OR² in General Formula (2). When R¹ includes a cycloalkylene group having -OR², the carbon-carbon bonds forming the ring may be converted into double bonds in Step (II) (described later).

In the present invention, R^{1'} is preferably, among the aforementioned rings, a group containing a bivalent 6-membered aromatic ring or a bivalent 6-membered heterocyclic aromatic ring, with a bonding site at the para-positions.

In the present invention, R^{1'} is preferably a group derived from a monocyclic or a condensed ring. A group derived from a monocyclic ring is more preferable. More specifically, the one or more other organic ring groups of R¹ are preferably a phenylene group (in particular, a p-phenylene group) and a naphthylene group (in particular, a 1,5-naphthylene group or 2,6-naphthylene group). A phenylene group (in particular, a p-phenylene group) is more preferable.

In General Formulae (1) and (3), n is an integer of 1 or more, preferably not more than 20, more preferably not more than 10, still more preferably not more than 5, and particularly preferably 1 or 2. When n is two or more, R¹ is two or more, and they may be the same or different.

In General Formula (4), as with n, n' is an integer of 1 or more, preferably not more than 20, more preferably not more than 10, still more preferably not more than 5, and particularly preferably 1 or 2. When n' is two or more, R^{1'} is two or more, and they may be the same or different. n' is the same as n in General Formula (3) that represents Compound (3), which is a starting material used in the present invention.

Further, m in General Formulae (1) and (4) is not particularly limited insofar as it is an integer not less than 2. m is generally not more than 10, preferably not more than 6, more preferably 2 to 4, and still more preferably 4.

### [1] Method for producing Cyclic Compound (1)

The method for producing Cyclic Compound (1) of the present invention comprises the step of:
(I) forming Compound (1) using Compounds (3) in the presence of a nickel compound.

In Step (I), a plurality of the same kind of Compounds (3) are coupled (homocoupling) to form Cyclic Compound (Z). This coupling reaction in Step (I) for reacting a plurality of the same Compounds (3) is a so-called Yamamoto coupling. Compound (3) has two halogen atoms, and the carbon atoms attached to these two halogen atoms in the plurality of Compounds (3), i.e., the carbon atoms attached to the halogen atoms in one of Compounds (3) and the carbon atoms attached to the two halogen atoms of the other one of Compounds (3) are coupled due to the presence of a nickel compound. In this manner, a coupling reaction of Compounds (3) is continuously advanced by coupling the carbon atoms therein, thereby obtaining Cyclic Compound (1).

Compound (3) is a compound represented by General Formula (3). Further, Compound (3) has a benzene ring having a halogen atom at each molecular terminus, and organic ring groups at least including a derivative group of an alicyclic hydrocarbon group represented by General Formula (2) (hereinafter, this may also be referred to as "a bivalent group represented by General Formula (2)"). In Compound (3), organic ring groups of a number corresponding to n in General Formula (3) are continuously coupled.

As shown in General Formula (3), Compound (3) used in the present invention is a compound made of phenylene groups having halogen atoms X, and a bivalent group represented by General Formula (2).

The benzene ring generally has a rigid plain structure. On the other hand, the cyclohexane ring of the bivalent group represented by General Formula (2) is attached to the benzene ring or the organic ring group at the 1-position and 4-position, forming a nonlinear (L-shaped) structure of chair conformation in which the benzene rings or the organic ring groups are respectively at axial and equatorial positions. Accordingly, the compound represented by General Formula (3) has a nonplanar or nonlinear structure, which differs from a linear structure.

Further, as described above, in addition to those having a chair conformation, there are cyclohexane rings having a nonplanar structure such as a boat conformation, a twist-boat conformation, or the like. Therefore, by suitably selecting a variety of organic ring groups, it becomes possible to obtain Compounds (3) of various structures.

For example, when Compound (3) has only one cyclohexylene derivative group having a chair conformation; and the rest of R¹ are planar organic ring groups, such as phenylene groups, Compound (3) is structured such that the cyclohexylene derivative group is bonded to the benzene rings or the organic ring groups at the 1-position and 4-position, forming a nonlinear (L-shaped) structure of chair conformation in which the benzene rings or the organic ring groups are at axial and equatorial positions. Further, in this L-shaped structure, the acute angle (hereinafter, "inner angle") in the corner of the cyclohexylene derivative group is substantially 90°. Further, when Compound (3) has two cyclohexane rings having a chair conformation as organic ring groups, Compound (3) has a U-shaped structure in which each inner angle is substantially 90°. Further, depending on the combination with other organic ring groups, Compound (3) may be designed into a V-Shaped structure, a W-shaped structure, and the like.

Further, in Step (I), a plurality of the same kind of Compounds (3) are coupled (homocoupling) to form Cyclic Compound (1). In this case, the number of bonds (number of couplings) in Compound (3) is not particularly limited, and generally depends on the structure of Compound (3). Therefore, the number of bonds (number of couplings) in Compound (3) is determined according to Compound (3) having a curved structure composed of cyclohexylene derivative groups represented by General Formula (2), or the like. For example, when Compound (3) has an L-shaped structure in which each inner angle is substantially 90°, Cyclic Compound (1) can be formed by coupling three or four Compounds (3), thereby obtaining a trimer or a tetramer of Compound (3). Further, when Compound (3) has a V-shaped structure in which each inner angle is substantially 60°, Cyclic Compound (1) can be formed by coupling three Compounds (3), thereby obtaining a trimer of Compound (3). Further, when Compound (3) has an L-shaped structure in which each inner angle is substantially 120°, Cyclic Compound (1) can be formed by coupling five or six Compounds (3), thereby obtaining a pentamer or a hexamer of Compounds (3). These are typical examples of homocoupling in Step (I); however, various reactions may be adopted insofar as they can form Cyclic Compound (1).

The carbon nanoring is made of a compound having a cyclic structure, such as a cycloparaphenylene. By using the compound having an L-shaped structure or the like as Compound (3), a compound having a cyclic structure can be easily formed. Therefore, by selecting an appropriate Compound (3) in the method of the present invention, it is possible to accurately select the number of organic ring groups to be coupled, thereby efficiently producing a carbon nanoring having a cyclic structure in which an arbitrary number of organic ring groups are continuously bonded, with a short production process.

Further, although there is no particular preference for Compound (3), a typical example thereof may be Compound (3a), as represented by General Formula (3a) below, having a cyclohexylene derivative group, and phenylene groups as the rest of the organic ring groups, 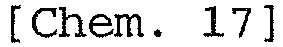 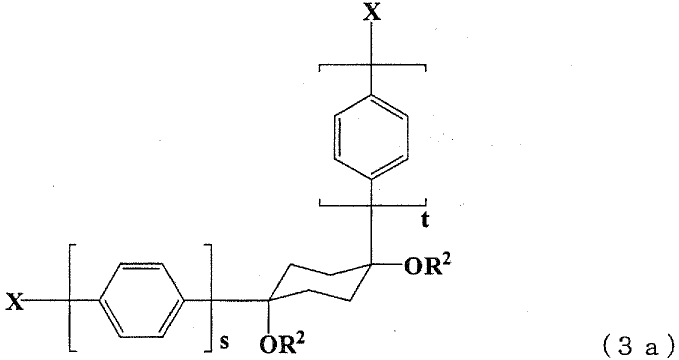 wherein X and R² are as defined above; s is an integer of 1 or more; t is an integer of 1 or more; and s+t = n+1 wherein n is as defined above.

In General Formula (3a), s and t are integers of 1 or more, preferably not more than 10, more preferably not more than 5, and still more preferably not more than 3, provided that the total of s and t is equal to n+1 (n is as defined in General Formula (3)).

A typical example of Compound (3a) is Compound (3a-1), as represented by General Formula (3a-1) below, wherein s and t are 1, 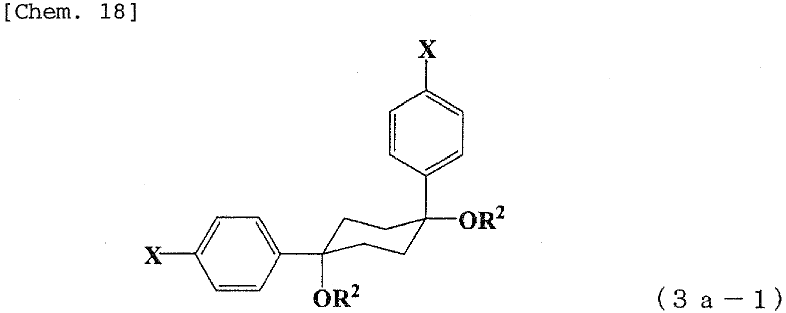 wherein X and R² are as defined above.

Further, Compound (3a) is not limited to compounds having an L-shaped structure, and a compound having a U-shaped structure may also be used. The compound having a U-shaped structure is not particularly limited, and examples thereof include Compound (3c) represented by General Formula (3c):

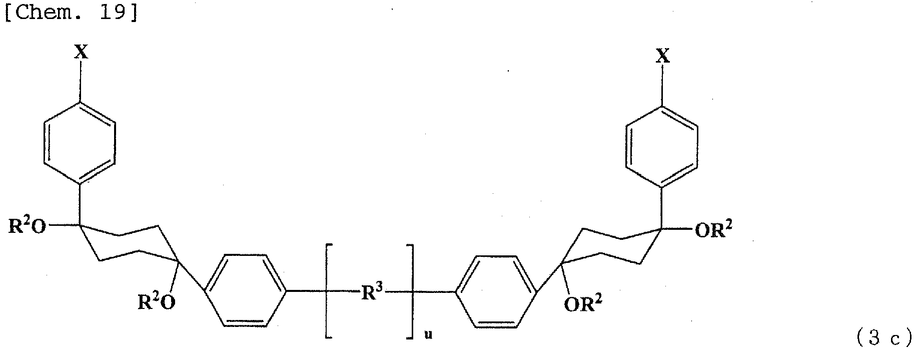

wherein X and R² are as defined above; u R³ are the same or different, and each represents a bivalent aromatic hydrocarbon group, a bivalent alicyclic hydrocarbon group, a bivalent heterocyclic group, or a derivative group thereof; u is an integer of 1 or more; and u = n-4.

R³ is the same as R^{1'}.

In General Formula (3c), u is an integer of 1 or more, preferably not more than 10, more preferably not more than 5, and still more preferably not more than 3, provided that u is identical to n-4 (n is as defined in General Formula (3)).

Examples of Compound (3c) include Compound (3c-1), as represented by General Formula (3c-1) below, wherein R³ is a p-phenylene group; and u is 1: 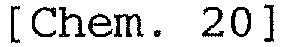 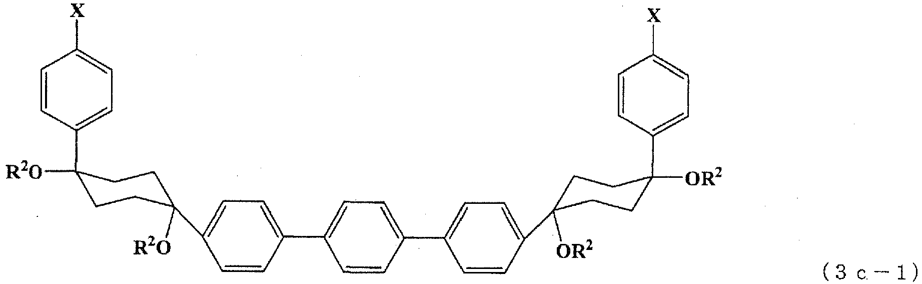 wherein X and R² are as defined above,
and Compound (3c-2), as represented by General Formula (3c-2) below, wherein R³ is a p-phenylene group; and u is 2: 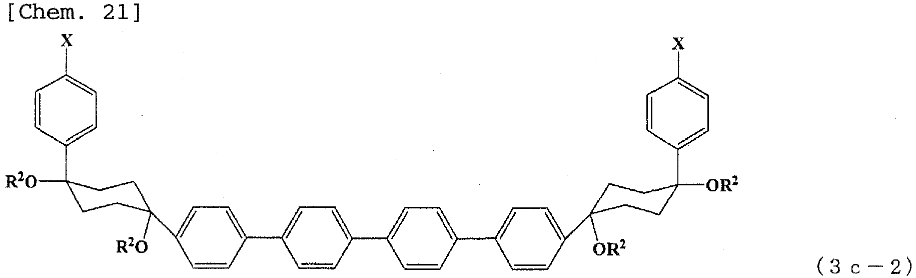 wherein X and R² are as defined above.

Step (I) of the present invention uses a nickel compound. The nickel compound is not particularly limited; however, zerovalent nickel salts or bivalent nickel salts are preferable. They may be used singly or in a combination of two or more. These complexes designate both a reagent to be added, and a product generated during the reaction.

The zerovalent nickel salts are not particularly limited, and examples thereof include bis(1,5-cyclooctadiene)nickel(0), bis(triphenylphosphine)nickeldicarbonyl, nickelcarbonyl, and the like.

Further, examples of bivalent nickel salts include nickel(II)acetate, nickel(II)trifluoroacetate, nickel(II)nitrate, nickel(II)chloride, nickel(II)bromide, nickel(II)acetylacetonato, nickel(II)perchlorate, nickel(II)citrate, nickel(II)oxalate, nickel cyclohexanebutyrate, nickel(II)benzoate, nickel(II)stearate, nickel(II)stearate, nickel(II)sulfamate, nickel(II)carbonate, nickel(II)thiocyanate, nickel(II)trifluoromethanesulfonate, bis(1,5-cyclooctadiene)nickel(II), bis(4-diethylamino dithiobenzyl)nickel (II), nickel(II) cyanide, nickel fluoride (II), nickel(II)boride, nickel(II)borate, nickel(II)hypophosphite, ammonium nickel(II)sulfate, nickel(II)hydroxide, nickel(II)cyclopentadienyl, hydrates thereof, and mixtures thereof.

Examples of zerovalent nickel salts and bivalent nickel salts also include compounds with previously coordinated ligands.

The amount of the nickel compound added as a reagent is generally 0.01 to 50 mol, preferably 0.1 to 10 mol, more preferably 0.5 to 5 mol, and particularly preferably 1 to 3 mol, per mol of Compound (3) used as a starting material.

In the production method of the present invention, a ligand that can be coordinated to nickel (a nickel atom) may be used as well as the nickel compound. Examples of such ligands include carboxylate-based ligands, amide-based ligands, phosphine-based ligands, oxime-based ligands, sulfonate-based ligands, 1,3-diketone-based ligands, Schiff base ligands, oxazoline-based ligands, diamine-based ligands, carbon monoxide ligands, carbene-based ligands, and the like. They may be used singly or in a combination of two or more. The coordinating atoms in the ligands are a nitrogen atom, phosphorus atom, and oxygen atom, sulfur atoms, and the like. These ligands include monodentate ligands having a coordinating atom at one site, and multidentate ligands having coordinating atoms at two or more sites. Further, in carbon monoxide ligands and carbene-based ligands, carbon atoms serve as coordinating atoms.

Examples of monodentate ligands include triphenylphosphine, trimethoxyphosphine, triethylphosphine, tri(i-propyl)phosphine, tri(tert-butyl)phosphine, tri(n-butyl)phosphine, tri(isopropoxy)phosphine, tri(cyclopentyl)phosphine, tri(cyclohexyl)phosphine, tri(orthotoluyl)phosphine, tri(mesityl)phosphine, tri(phenoxy)phosphine, tri-(2-furyl)phosphine, bis(p-sulfonatophenyl)phenylphosphine potassium, di(tert-butyl)methylphosphine, methyldiphenylphosphine, dimethylphenylphosphine, triethylamine, pyridine, and the like.

Examples of bidentate ligands include 2,2'-bipyridine, 4,4'-(tert-butyl)bipyridine, phenanthroline, 2,2'-bipyrimidyl, 1,4-diazabicyclo[2,2,2]octane, 2-(dimethylamino)ethanol, tetramethylethylenediamine, N,N-dimethylethylenediamine, N,N'-dimethylethylenediamine, 2-aminomethylpyridine, or (NE)-N-(pyridine-2-ylmethyliden)aniline, 1,1'-bis(diphenylphosphino)ferrocene, 1,1'-bis(tert-butyl)ferrocene, diphenylphosphino methane, 1,2-bis(diphenylphosphino)ethane, 1,3-bis(diphenylphosphino)propane, 1,5-bis(diphenylphosphino)pentane, 1,2-bis(dipentafluorophenylphosphino)ethane, 1,2-bis(dicyclohexylphosphino)ethane, 1,3-(dicyclohexylphosphino)propane, 1,2-bis(di-tert-butylphosphino)ethane, 1,3-bis(di-tert-butylphosphino)propane, 1,2-bis(diphenylphosphino)benzene, 1,5-cyclooctadiene, BINAP, BIPHEMP, PROPHOS, DIOP, DEGUPHOS, DIPAMP, DuPHOS, NORPHOS, PNNP, SKEWPHOS, BPPFA, SEGPHOS, CHIRAPHOS, JOSIPHOS, and mixtures thereof.

Further, examples of BINAP include BINAP derivatives. Examples thereof include 2,2'-bis(diphenylphosphino)-1,1'-binaphthyl, 2,2'-bis(di-p-tolylphosphino)-1,1'-binaphthyl, 2,2'-bis(di-p-tertiary-butylphenylphosphino)-1,1'-bi naphthyl, 2,2'-bis(di-m-tolylphosphino)-1,1'-binaphthyl, 2,2'-bis(di-3,5-dimethylphenylphosphino)-1,1'-binaphthyl, 2,2'-bis(di-p-methoxyphenylphosphino)-1,1'-binaphthyl, 2,2'-bis(dicyclopentylphosphino)-1,1'-binaphthyl, 2,2'-bis(dicyclohexylphosphino)-1,1'-binaphthyl, 2-di(β-naphthyl)phosphino-2'-diphenylphosphino-1,1'-binaphthyl, 2-diphenylphosphino-2'-di(p-trifluoromethylphenyl)phosphino-1,1'-binaphthyl, and the like.

Further, examples of BIPHEMP include BIPHEMP derivatives. Examples thereof include 2,2'-dimethyl-6,6'-bis(diphenylphosphino-1,1'-biphenyl, 2,2'-dimethyl-6,6'-bis(dicyclohexylphosphino)-1,1'-biphenyl, 2,2'-dimethyl-4,4'-bis(dimethylamina)-6,6'-bis(diphenylphosphino)-1,1`-biphenyl, 2,2',4,4'-tetramethyl-6,6'-bis(diphenylphosphino)-1,1'-biphenyl, 2,2'-dimethoxy-6,6'-bis(diphenylphosphino)-1,1'-biphenyl, 2,2',3,3'-tetramethoxy-6,6'-bis(diphenylphosphino)-1,1'-biphenyl, 2,2',4,4'-tetramethyl-3,3'dimethoxy-6,6'-bis(diphenylphosphino)-1,1'-biphenyl, 2,2'-dimethyl-6,6'-bis(di-p-tolylphosphino)-1,1'-biphenyl, 2,2'-dimethyl-6,6'-bis(di-p-tert-butylphenylphosphino)-1,1'-biphenyl, and 2,2',4,4'-tetramethyl-3,3'-dimethoxy-6,6'-bis(di-p-methoxyphenylphosphino)-1,1'-biphenyl.

When these ligands are used, the amount thereof is generally, 0.01 to 50 mol, preferably 0.1 to 10 mol, more preferably 0.5 to 5 mol, and particularly preferably 1 to 3 mol, per mol of Compound (3).

The reaction in Step (I) is generally performed in the presence of a reaction solvent. Examples of reaction solvents include aliphatic hydrocarbons (hexane, cyclohexane, heptane, and the like), aliphatic halogenated hydrocarbons (dichloromethane, chloroform, carbon tetrachloride, dichloroethane, and the like), aromatic hydrocarbons (benzene, toluene, xylene, chlorobenzene, and the like), ethers (diethylether, dibutylether, dimethoxyethane(DME), cyclopentylmethylether (CPME), tert-butylmethylether, tetrahydrofuran, dioxane, and the like), esters (ethyl acetate, ethyl propanoate, and the like), acid amides (dimethylformamide (DMF), dimethylacetamido (DMA), N-methylpyrrolidone (1-methyl-2-pyrrolidone) (NMP), and the like), nitriles (acetonitrile, propionitrile, and the like), and dimethylsulfoxides (DMSO). They may be used singly or in a combination of two or more.

In Step (I), the amount of the solvent is generally 1 to 1,000 parts by mass, preferably 5 to 200 parts by mass, and more preferably 10 to 100 parts by mass, per 100 parts by mass of Compound (3).

In Step (I), the reaction temperature is generally not less than 0°C and not more than the boiling point of the reaction solvent.

The reaction atmosphere is not particularly limited; however, an inert gas atmosphere, such as an argon gas atmosphere or a nitrogen gas atmosphere, is preferable. An air atmosphere may also be adopted.

In Step (I), a plurality of identical Compounds (3) are coupled, thereby producing Cyclic Compound (1) represented by General Formula (1): 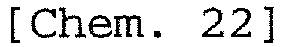 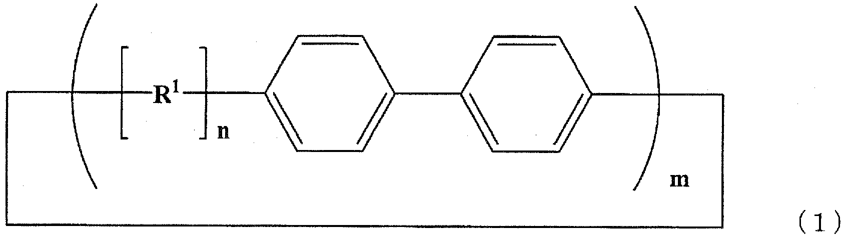 wherein R¹, n and m are as defined above.

Compound (1) is a cyclic compound in which R¹ as organic ring groups and phenylene groups are continuously bonded. As mentioned above, because the number of n and m in General Formula (1) can be adjusted depending on the shape and the number of rings of Compound (3), it is possible to adjust the total number of R¹ and phenylene groups in Cyclic Compound (1).

In Step (I), when the compound represented by General Formula (3a) is used as Compound (3), generally, a tetramer in which four Compounds (3a) are coupled, i.e., Cyclic Compound (1a) represented by Formula (1a) below is obtained, 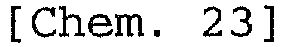 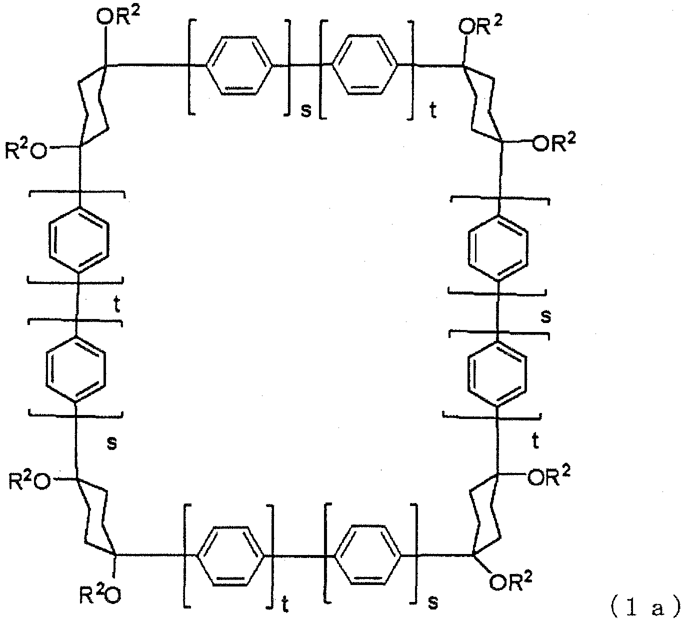 wherein R², s and t are as defined above.

### [2] Method for producing Carbon Nanoring (4)

The method for producing Carbon Nanoring (4) of the present invention comprises, after Step (I) for producing Cyclic Compound (1), Step (II) for converting cyclohexane rings of Cyclic Compound (1) into benzene rings.

For example, this step may be performed by a general oxidation reaction. As concrete examples, a method of heating Cyclic Compound (1) in the presence of acid (acid-treatment), a method of heating the compound in the presence of oxygen (in air atomosphere, oxygen atmosphere, etc.), a reaction with quinones, metallic oxides, etc., may be adopted. Such processes are generally performed by a dehydrogenation reaction, or the like, thereby chemically changing (aromatizing) the cyclohexane rings of Cyclic Compound (1) into benzene rings to synthesize Carbon Nanoring (4). More specifically, the above processes eliminate the OR² that exists in each cyclohexane ring of the cyclic compound before conversion, while advancing the dehydrogenation reaction, thereby yielding Carbon Nanoring (4).

More specifically, by converting cyclohexane rings of Cyclic Compound (1) obtained in Step (I) into benzene rings, it is possible to obtain Carbon Nanoring (4) represented by General Formula (4): 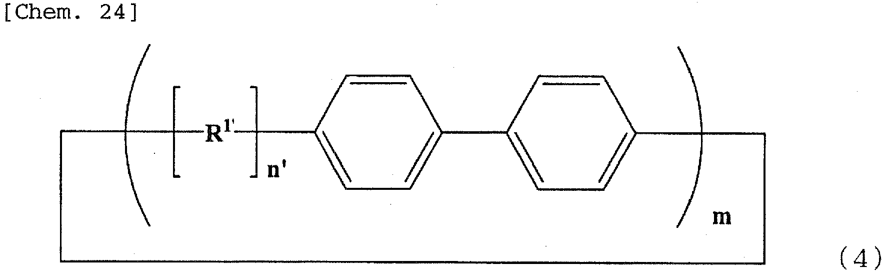 wherein R^{1'}, n' and m are as defined above.

Further, the cyclohexane rings in Compound (1) derived from Compound (3) may be converted into phenylenes through a dehydrogenation reaction, or the like. In particular, because the cyclohexylene derivative group in General Formula (2) has a structure in which OR², such as a hydroxy group, is disposed at each of the 1-position and 4-position of the cyclohexylene, it is possible to more efficiently convert these derivative groups into phenylenes.

For example, when Compound (1a) is obtained as Compound (1) in Step (I), it is possible to obtain Compound (4a) below in Step (II) by converting the cyclohexane rings in Compound (1) into benzene rings, 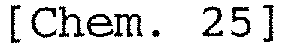 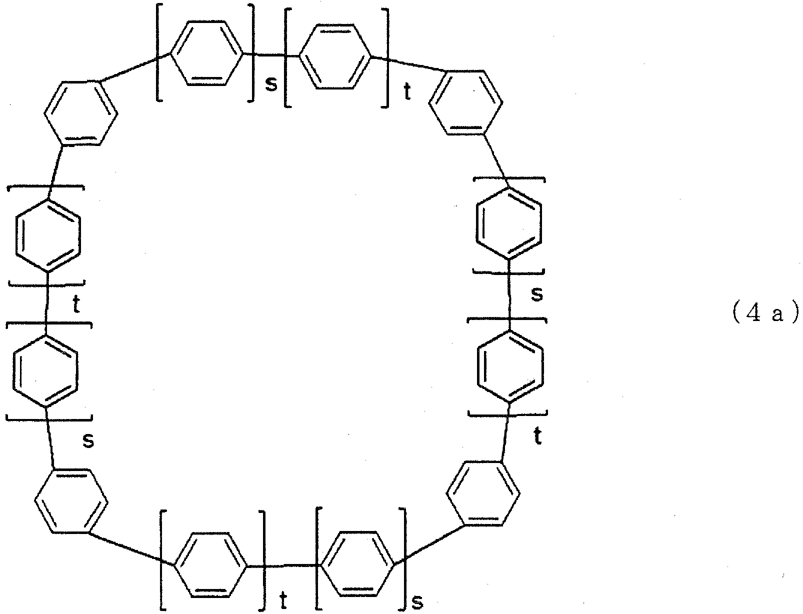 wherein, s and t are as defined above.

Step (II) is preferably performed by subjecting Cyclic Compound (1) to heat treatment in the presence of an acid (hereinafter, "acid treatment"), thereby chemically changing the cyclohexane rings of Cyclic Compound (1) into benzene rings.

The method of the above acid treatment is not particularly limited. Examples thereof include the following methods.
(A) A method of dissolving Cyclic Compound (1) and an acid in a solvent, and reacting the resulting solution by heating.
(B) A method of dissolving Cyclic Compound (1) in a solvent, mixing the resulting solution with an acid, and reacting the resulting mixture by heating.

In Step (II), the acid treatment can be performed without a solvent.

The acid used in Methods (A) and (B) above is not particularly limited; however, strong acids used for catalysts or the like are preferable. Examples thereof include sulfuric acids, methanesulfonic acids, para-toluenesulfonic acids, tungstophosphoric acids, tungstosilicic acids, molybdophosphoric acids, molybdosilicic acids, boron trifluoride etherates, sodium hydrogen sulfate, and tin tetrachlorides. They may be used singly or in a combination of two or more.

The acid amount may be varied depending on the production conditions, etc. However, in Method (A) above, the acid amount is preferably 0.01 to 100 molar equivalents, more preferably 0.5 to 50 molar equivalents, and yet more preferably 1 to 20 molar equivalents, relative to Cyclic Compound (1).

In Method (B) above, the acid amount is preferably 0.01 to 100 molar equivalents, more preferably 0.5 to 50 molar equivalents, and yet more preferably 1 to 20 molar equivalents, relative to Cyclic Compound (1).

Both nonpolar solvents and polar solvents may be used as solvents for the acid treatment reaction. Examples thereof include alkanes such as hexane, heptane, or octane; haloalkanes such as methylene chloride, chloroform, carbon tetrachloride, or ethylene chloride; benzenes such as benzene, toluene, xylene, mesitylene, or pentamethylbenzene; halobenzenes such as chrolobenzene or bromobenzene; ethers such as diethyl ether or anisole; and dimethylsulfoxides. These solvents may be used singly or in a combination of two or more. In the reaction using a solvent, the reaction intermediate between Cyclic Compound (1) and the carbon nanoring may have low solubility with respect to the Solvent 1 used in the above Step 1. In this case, another solvent may be added in advance or during the reaction.

When a solvent is used, the amount thereof is appropriately determined depending on the production conditions. However, it is preferable that the amount of solvent be 100 to 100,000 parts by mass, and more preferably 1,000 to 10,000 parts by mass, based on 100 parts by mass of Cyclic Compound (1).

The heating temperature in Methods (A) and (B) above is generally 50°C or more, preferably 80°C or more, more preferably 100°C or more, and yet more preferably 120°C or more. When a solvent is used, the temperature is set in a range of not more than the boiling point of the solvent.

The heating is performed by using, for example, an oil bath, an aluminum block constant-temperature bath, a heat gun, a burner, microwave irradiation, etc. In the case of microwave irradiation, it is possible to use a known microwave reaction device for microwave reaction. Reflux cooling may be performed together with the heating process.

The reaction atmosphere in the acid treatment is not particularly limited; however, an inert gas atmosphere, such as an argon gas atmosphere or a nitrogen gas atmosphere, is preferable. An air atmosphere may also be adopted.

Further, the method for producing the carbon nanoring of the present invention may also include a purification step after Step (II) as necessary. More specifically, general post-treatment steps, such as solvent removal, washing, chromatography separation, or the like, may be performed. In particular, because Carbon Nanoring (1) resulting from Step (II) is usually amorphous, the nanoring can be crystallized using a hitherto-known recrystallization method for organic compounds. In the resulting crystal, the organic solvent used for the recrystallization may be incorporated in the ring of the molecule.

The carbon nanoring obtained by the method for producing carbon nanorings of the present invention is made of a compound having a cyclic structure in which organic ring groups, which are at least one member selected from the group consisting of bivalent aromatic hydrocarbon groups, bivalent alicyclic hydrocarbon groups, bivalent heterocyclic groups, etc., are continuously bonded. The method of the present invention enables the production of a carbon nanoring in which 9 or more, in particular, 12 or more organic ring groups are continuously bonded. The number of organic ring groups is not particularly limited; however, it is generally not more than 100, preferably not more than 50, more preferably not more than 30, and yet more preferably not more than 20.

As explained above, the production method of the present invention enables the production of carbon nanorings from various types of Compound (3). Therefore, the resulting Carbon Nanoring (4) has a structure such that the hydrogen atoms bonded to the carbon atoms of the organic ring contained in the cyclic compound are substituted with functional groups.

Further, the diameter of the carbon nanorings (4) is about 1.8 to 2.4 nm when the nanoring has 12 to 16 organic ring groups (in particular, a phenylene group). Further, the diameter is about 1.2 to 2.4 nm when the nanoring has 9 to 16 organic ring groups (in particular, a phenylene group).

Further, in the carbon nanorings (4), it is preferable that at least 8 organic ring groups are derived from aromatic rings. It is preferable that all of the organic ring groups be aromatic hydrocarbon groups. It is more preferable that the carbon nanorings (4) be made of compounds in which all of the organic ring groups are phenylene groups (in particular, p-phenylene groups).

Further, in the carbon nanorings (4), if all of the organic ring groups are phenylene groups, it is particularly preferable that the compound be a cycloparaphenylene compound having 9 or more, in particular, 12 or more phenylene groups. In this cycloparaphenylene compound, the phenylene groups are preferably directly linked at the 1-positions and 4-positions.

The cycloparaphenylene compound containing a specific number of phenylene groups is useful as a raw material for synthesizing (pure synthesis) a carbon nanotube having a specific radius range. Such a cycloparaphenylene compound is also suitable for electronic industry materials, luminescence materials, and the like.

### [3] Methods for producing starting materials

A method for producing Compounds (3) used in the present invention is described below.

### Compound (3a-1)

The compound represented by General Formula (3a-1) above can be obtained, for example, by reacting 1,4-cyclohexanedione represented by Formula (6) below: 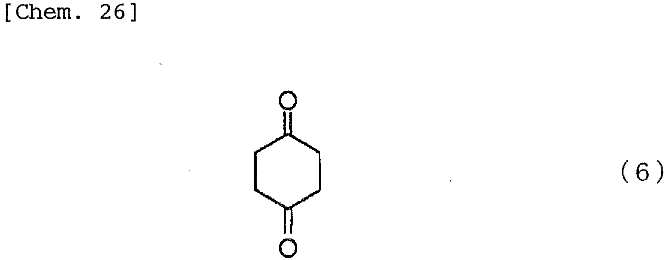 with a compound (which may also be referred to as an "aromatic dihalogen compound" hereinafter) represented by General Formula (7): 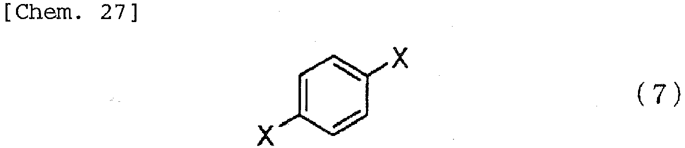 wherein X is as defined above.

The aromatic dihalogen compound represented by General Formula (7) is not particularly limited insofar as the aromatic dihalogen compound has halogen atoms at the 1-position and 4-position. Examples of such compounds include 1,4-dibromobenzene, 1,4-diiodobenzene, and 1-bromo-4-iodobenzene.

When the compound represented by General Formula (3a-1) as Compound (3) is produced in the above method, the amounts of 1,4-cyclohexanedione and the aromatic dihalogen compound represented by General Formula (7) are as follows. The amount of the aromatic dihalogen compound is preferably 2.0 to 10 mol, more preferably 2.3 to 5.0 mol, and still more preferably 2.5 to 3.5 mol, per mol of 1,4-cyclohexanedione.

The method using the above raw material is not particularly limited. More specifically, the following method can be adopted. The aromatic dihalogen compound is reacted with an organic alkali metal compound to cause an interchange reaction of an alkali metal with a halogen atom, thereby obtaining a precursor compound containing a halogen atom and a hydrocarbon group in which a halogen atom of the aromatic dihalogen compound is substituted with a hydrocarbon group of the organic alkali metal compound; then, the resulting precursor compound is reacted with 1,4-cyclohexanedione to cause a nucleophilic addition reaction, thereby obtaining the compound represented by General Formula (3a-1).

Examples of the organic alkali metal compounds include organic lithium compounds and organic sodium compounds. Organic lithium compounds are particularly preferable. Examples of organic lithium compounds include organic monolithium compounds, organic dilithium compounds, and organic polylithium compounds.

Examples of organic lithium compounds include ethyllithium, n-propyllithium, isopropyllithium, n-butyllithium, sec-butyllithium, tert-butyllithium, pentyllithium, hexyllithium, cyclohexyllithium, phenyllithium, hexamethylene dilithium, cyclopentadienyl lithium, indenyl lithium, 1,1-diphenyl-n-hexyllithium, 1,1-diphenyl-3-methylpentyllithium, lithium naphthalene, butadienyl dilithium, isopropenyl dilithium, m-diisoprenyl dilithium, 1,3-phenylene-bis-(3-methyl-1-phenylpentylidene)bislithium, 1,3-phenylene-bis-(3-methyl-1,[4-methylphenyl] pentylidene)bislithium, 1,3-phenylene-bis-(3-methyl-1,[4-dodecylphenyl] pentylidene)bislithium, 1,1,4,4-tetraphenyl-1,4-dilithio butane, polybutadienyl lithium, polyisoprenyl lithium, polystyrene butadienyl lithium, polystyrenyl lithium, polyethylenyl lithium, poly-1,3-cyclohexa dienyl lithium, polystyrene 1,3-cyclohexadienyl lithium, and polybutadiene 1,3-cyclohexadienyl lithium. Among these, n-butyllithium is preferable.

The amount of organic alkali metal compound is preferably 0.8 to 5 mol, more preferably 0.9 to 3.0 mol, and still more preferably 0.9 to 1.2 mol, per mol of the aromatic dihalogen compound represented by General Formula (7).

In the above method for producing the compound represented by General Formula (3a-1), the following combinations are particularly preferred. 1,4-diiodobenzene and 1,4-dibromobenzene are used as the aromatic dihalogen compound, and n-butyllithium is used as the organic alkali metal compound. When 1,4-diiodobenzene is used as the aromatic dihalogen compound, the reaction of 1,4-diiodobenzene with n-butyllithium produces 4-iodophenyllithium. Then, by causing a nucleophilic addition reaction of 4-iodophenyllithium and cyclohexane 1,4-dione, the compound represented by Formula (3a-1a) below: 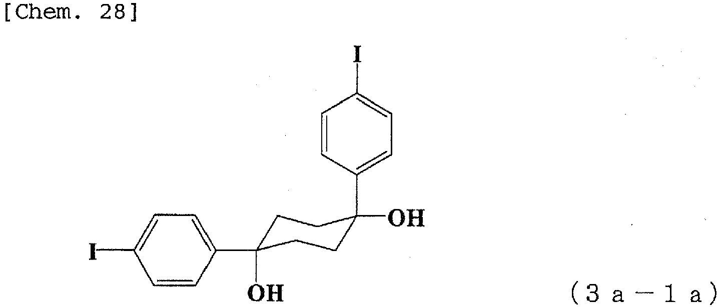 is obtained.
This compound is Compound (3a-1).

The reaction of the aromatic dihalogen compound and the organic alkali metal compound is generally performed in the presence of a reaction solvent. The reaction solvent can be selected from the aforementioned reaction solvents used in the above coupling reaction step of the present invention.

The temperature in the reaction of the aromatic dihalogen compound and the organic alkali metal compound is generally selected from a range not less than 0°C and not more than the boiling point of the reaction solvent.

Further, the reaction atmosphere is not particularly limited; however, an inert gas atmosphere, such as an argon gas atmosphere, a nitrogen gas atmosphere, etc., is preferable. It is also possible to adopt an air atmosphere.

### Compound (3a)

Further, by reacting the compound represented by General Formula (3a-1)(more specifically, General Formula (3a-1a)) and other compounds, it is possible to obtain other kinds of Compound (3).

For example, by reacting the compound represented by General Formula (8): 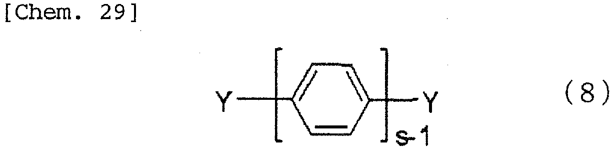 wherein, Y is a halogen atom or a group represented by General Formula (9): 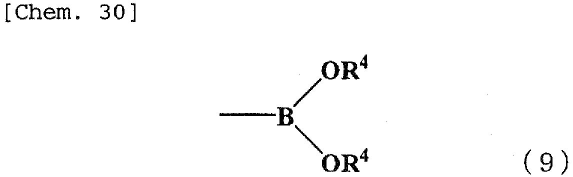 wherein R⁴ is the same or different, and each represents a hydrogen atom or a C₁-C₁₀ alkyl group; the group represented by General Formula (9) is a monovalent boronic acid or an ester thereof; at least one of the Ys is a halogen atom; when the two Ys are both halogen atoms, they may be the same or different; and s is as defined above,
or
the compound represented by General Formula (10): 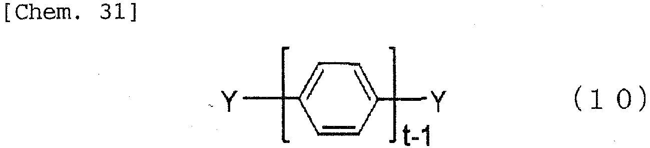 wherein Y and t are as defined above, with the compound represented by General Formula (3a-1), it is possible to obtain the compound represented by General Formula (3a).

In General Formulae (8) and (10), Examples of the halogen atom of Y include a fluorine atom, chlorine atom, bromine atom, and iodine atom.

Further, the group represented by General Formula (9) is a monovalent boronic acid or an ester thereof. When Y in General Formulae (8) and (10) is a group represented by General Formula (9), it is possible to bond them with Compound (3a-1) through a so-called Suzuki coupling.

Examples of the groups represented by General Formula (9) include the groups represented by Formulae (9a) to (9c) below. When these groups represented by Formulae (9a) to (9c) are used as the group represented by General Formula (9), reactions to form various Compounds (3) by coupling the compound represented by General Formula (3a-1) with the compound represented by General Formula (8) or (10) can be efficiently performed.

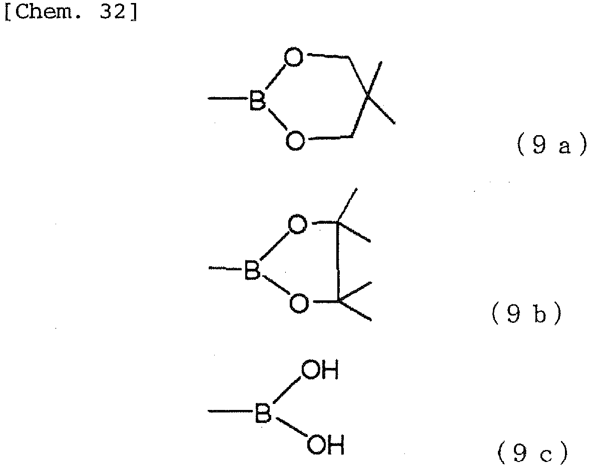

### Compound (3c)

Compound (3c) is obtained through the scheme represented by Reaction Formula 2: 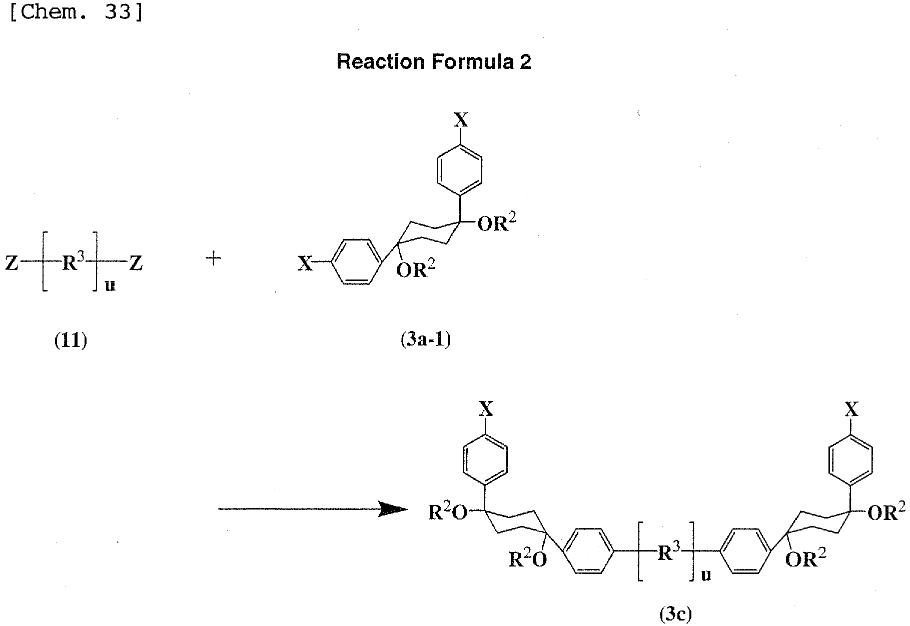 wherein X, R², R³ and u are as defined above; Z is the same or different, and each represents a group represented by General Formula (9): 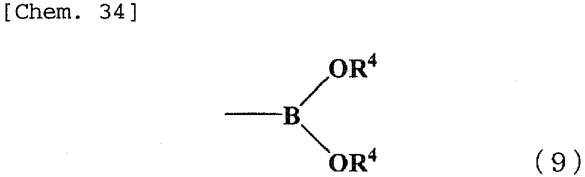 wherein R⁴ is as defined above.

This reaction in Reaction Formula 2 is described below.

Compound (3c) is obtained by reacting a raw material containing Compound (3a-1) and Compound (11) represented by General Formula (11): 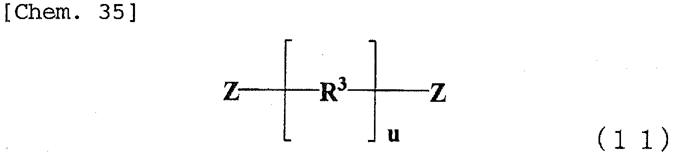 wherein Z, R³ and u are as defined above,
in the presence of a palladium catalyst.

This reaction of Compound (3a-1) with Compound (11) can be performed using a Suzuki-Miyaura coupling reaction.

Suzuki-Miyaura coupling is performed using a catalyst. The above reaction is also performed using a catalyst. In the present invention, a palladium catalyst is preferable.

Further, Z in General Formula (11) is a monovalent boronic acid represented by General Formula (9) or an ester thereof. In General Formula (11), the two Z groups may be the same or different. The two R⁴ groups in General Formula (9) may be the same or different. Further, when R⁴ is an alkyl group, the carbon atoms of these alkyls may be bonded to form a ring with the boron atom and the oxygen atoms.

Examples of Z in General Formula (11) include the groups represented by Formulae (9a) to (9c) below.

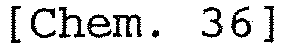

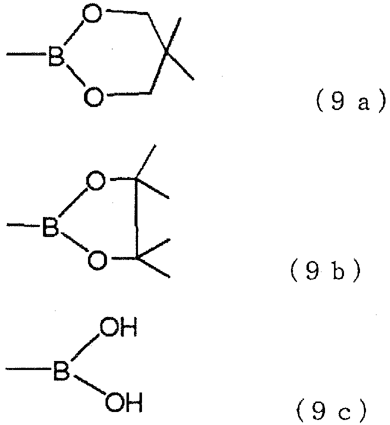

When these groups represented by Formulae (9a) to (9c) are used as Z in General Formula (11), the reaction of Compound (3a-1) with Compound (11) can be more efficiently performed.

The amounts of Compound (3a-1) and Compound (11) in the above reaction step are as follows in view of the yield of Compound (3c). The amount of Compound (11) is preferably 0.01 to 0.5 mol, more preferably 0.05 to 0.4 mol, and more preferably 0.08 to 0.2 mol, per mol of Compound (3a-1).

In the above reaction step, as explained, the reaction is generally performed in the presence of a palladium catalyst. Examples of palladium catalysts include metallic palladium and various known palladium compounds to be used as a catalyst for synthesizing organic compounds (including polymer compounds), etc. In the present invention, the palladium catalysts (palladium compounds) usable in a Suzuki-Miyaura coupling reaction may be used. Specific examples thereof include Pd(PPh₃)₄ (Ph represents a phenyl group), PdCl₂(PPh₃)₂ (Ph represents a phenyl group), Pd(OAc)₂ (Ac represents an acetyl group), tris(dibenzylideneacetone)dipalladium(0)(Pd₂(dba)₃), tris(dibenzylideneacetone)dipalladium(0)chloroform complex, bis(dibenzylideneacetone)palladium(0), bis(tri-t-butyl)phosphino)palladium(0), and (1,1'-bis(diphenylphosphino)ferrocene)dichloropalladium(II). Among them, Pd(PPh₃)₄ is preferable.

The amount of the palladium catalyst is generally 0.0001 to 0.1 mol, preferably 0.0005 to 0.02 mol, and more preferably 0.001 to 0.01 mol, per mol of Compound (3a-1), in terms of the yield.

Further, in the above reaction step, as required, it is possible to use a phosphorus ligand that can be coordinated with the palladium atom that is the center element of the palladium catalyst. Examples of phosphorus ligands include triphenylphosphine, tri-o-tolylphosphine, tri-m-tolylphosphine, tri-p-tolylphosphine, tris(2,6-dimethoxyphenyl)phosphine, tris[2-(diphenylphosphino)ethyl]phosphine, bis(2-methoxyphenyl)phenylphosphine, 2-(di-*t*-butylphosphino)biphenyl, 2-(dicyclohexylphosphino)biphenyl, 2-(diphenylphosphino)-2'-(N,N-dimethylamino)biphenyl, tri-*t*-butylphosphine, bis(diphenylphosphino)methane, 1,2-bis(diphenylphosphino)ethane, 1,2-bis(dimethylphosphino)ethane, 1,3-bis(diphenylphosphino)propane, 1,4-bis(diphenylphosphino)butane, 1,5-bis(diphenylphosphino)pentane, 1,6-bis(diphenylphosphino)hexane, 1,2-bis(dimethylphosphino)ethane, 1,1'-bis(diphenylphosphino)ferrocene, bis(2-diphenylphosphinoethyl)phenylphosphine, 2-(dicyclohexylphosphino-2',6'-dimethoxy-1,1'-biphenyl(S-Phos), 2-(dicyclohexylphosphino-2',4',6'-tri-isopropyl-1,1'-biphenyl(X-Phos), and bis(2-diphenylphosphinophenyl)ether(DPEPhos). In the present step, 2-(dicyclohexylphosphino)-2', 4',6'-tri-isopropyl-1,1'-biphenyl(X-Phos), and the like, are preferable.

When a phosphorus ligand is used in the above reaction step, the amount is, in terms of the yield, generally 0.01 to 1.0 mol, preferably 0.01 to 0.8 mol, and more preferably 0.05 to 0.3 mol, per mol of Compound (3a-1).

Further, in the reaction step, a base (a reagent for activation of boron species) may be used in addition to the palladium catalyst. This base is not particularly limited insofar as the base is a compound that can form an ate complex on the boron atom by a Suzuki-Miyaura coupling reaction. More specifically, examples of bases include potassium fluoride, cesium fluoride, sodium hydroxide, potassium hydroxide, sodium methoxide, sodium hydrogen carbonate, potassium hydrogen carbonate, sodium carbonate, potassium carbonate, cesium carbonate, potassium phosphate, sodium acetate, potassium acetate, and calcium acetate. Among these, cesium fluoride, cesium carbonate and potassium phosphate are preferable. The amount of the base (activating agent) is generally about 0.01 to 10 mol, preferably 0.1 to 5.0 mol, and more preferably 0.5 to 1.0 mol, per mol of Compound (3a-1).

Further, the above reaction is generally performed in the presence of a reaction solvent. Examples of reaction solvents include aromatic hydrocarbons such as toluene, xylene, or benzene; esters such as methyl acetate, ethyl acetate, or butyl acetate; cyclic ethers such as diethylether, tetrahydrofuran, dioxane, dimethoxyethane, or diisopropylether; halogenated hydrocarbons such as methyl chloride, chloroform, dichloromethane, dichloroethane, or dibromoethane; ketones such as acetone or methylethylketone; amides such as dimethylformamide or dimethylacetamide; nitriles such as acetonitrile; alcohols such as methanol, ethanol, or isopropylalcohol; and dimethylsulfoxides. These substances can be used singly or in a combination of two or more. Among them, tetrahydrofuran, etc., are preferable in the present invention.

The reaction temperature in the above reaction step is generally not less than 0°C, and is selected from a temperature range not more than the boiling point of the reaction solvent.

Further, the reaction atmosphere in the above reaction step is not particularly limited; however, an inert gas atmosphere, such as an argon gas atmosphere, a nitrogen gas atmosphere, etc., is preferable. It is also possible to adopt an air atmosphere.

Further, in the process of producing Compound (3c), a purification step may be performed after the reaction step as necessary. Specifically, general post-treatment steps, such as solvent removal (when a solvent is used), washing, chromatography separation, or the like, may be performed.

Compound (3c) can be formed by reacting Compound (3a-1) and Compound (11). Therefore, by appropriately setting the number "u" of R³ in Compound (11), it is possible to arbitrarily and accurately design the number of organic rings, i.e., the length of Compound (3c). This enables accurate designing of the length of Compound (3c).

### [4] Embodiments

Embodiments of the present invention are shown below.

### (a) A carbon nanoring having 12 p-phenylene groups

By using Compound (3a-1) represented by General Formula (3a-1): 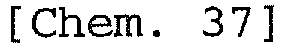 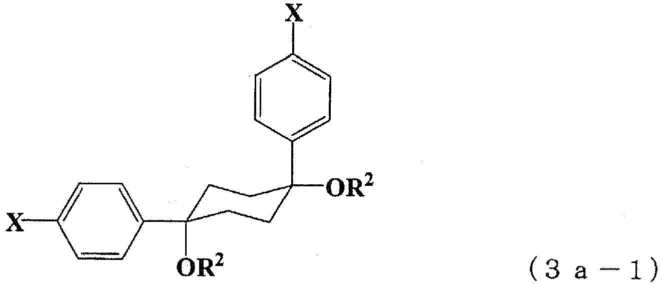 wherein X and R² are as defined above,
as Compound (3), i.e., as a starting material for a carbon nanoring, a tetramer is obtained.

Thereby, Cyclic Compound (1a-1) represented by General Formula (1a-1): 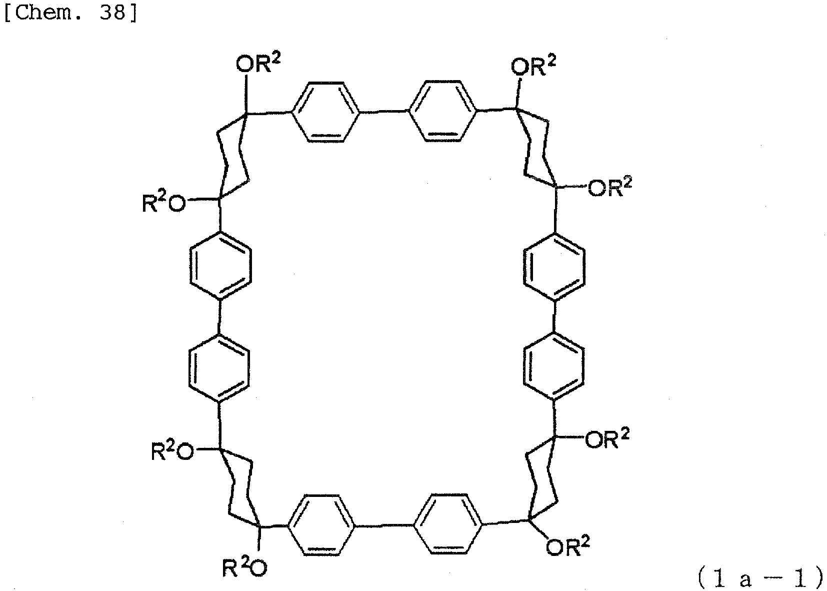 wherein R² is as defined above,
is obtained.

Cyclic Compound (1a-1) is an example of Cyclic Compound (1) in which R¹ is a bivalent group represented by General Formula (2): 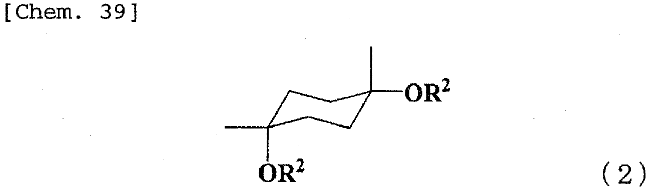 wherein R² is as defined above;
n is 1; and m is 4.

Further, by subjecting Cyclic Compound (1a-1) to Step (II), a cycloparaphenylene compound (which hereinafter may also be referred to as "[12]cycloparaphenylene") that has 12 p-phenylene groups and is represented by the following Formula (4a-1): 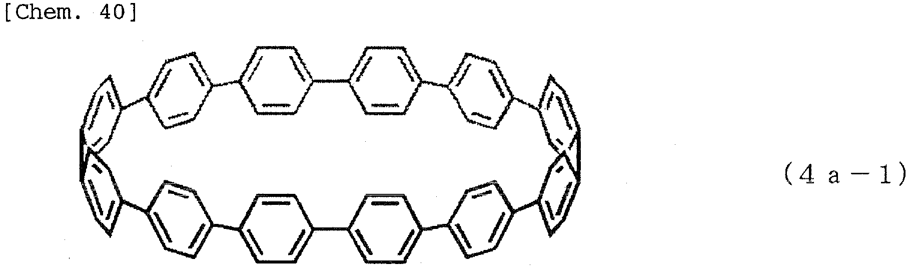 is obtained.

More specifically, a carbon nanoring made of [12]cycloparaphenylene can be easily produced using the compound represented by General Formula (3a-1).

### (b) A carbon nanoring having 9 p-phenylene groups

By using Compound (3a-1) represented by General Formula (3a-1): 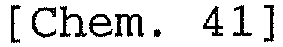 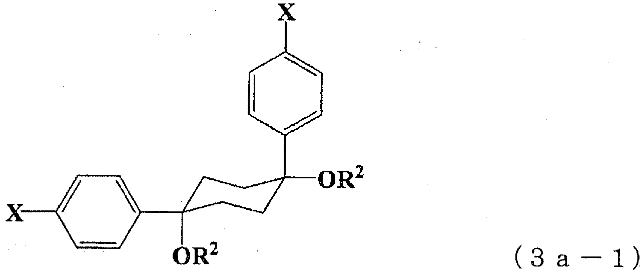 wherein X and R² are as defined above,
as Compound (3), i.e., as a starting material for a carbon nanoring, not only a tetramer but also a trimer is obtained.

Thereby, Cyclic Compound (1b) represented by General Formula (1b): 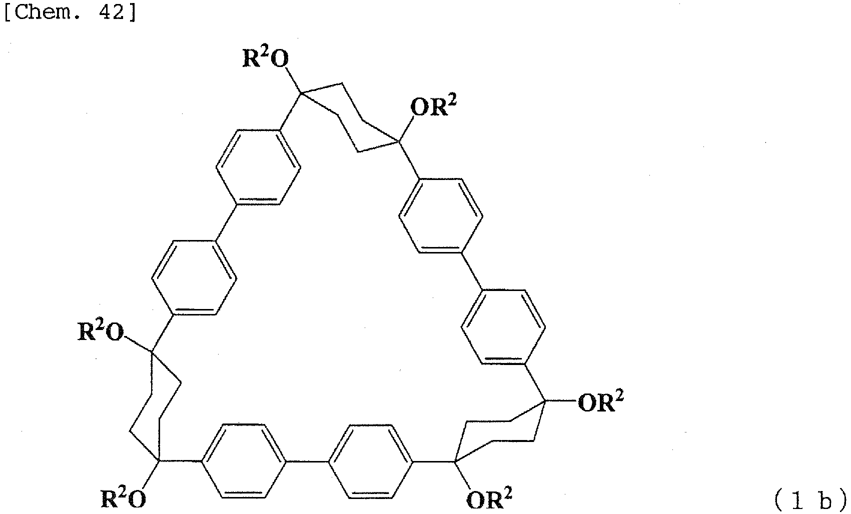 wherein R² is as defined above,
is obtained.

Cyclic Compound (1b) is an example of Cyclic Compound (1) in which R¹ is a bivalent group (2) represented by General Formula (2): 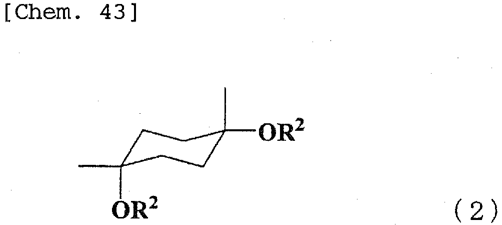 wherein R² is as defined above;
n is 1; and m is 3.

Cyclic Compound (1b) is a novel compound that has never been publically disclosed.

Further, by subjecting Cyclic Compound (1b) to Step (II), a cycloparaphenylene compound (which hereinafter may also be referred to as "[9]cycloparaphenylene") that has 9 p-phenylene groups and is represented by the following Formula (4b): 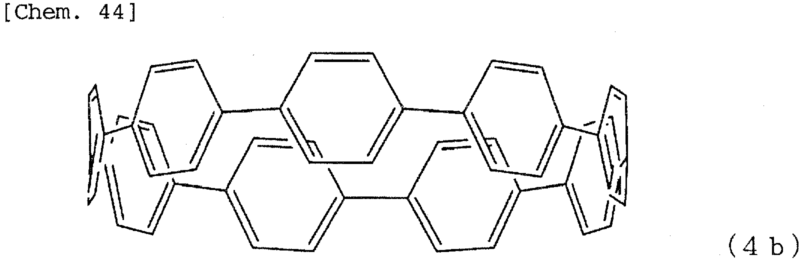 is obtained.

More specifically, the carbon nanoring made of [9]cycloparaphenylene can be easily produced using the compound represented by General Formula (3a-1).

As such, by using Compounds (3a-1) as a starting material, a mixture of trimers and tetramers is obtained; however, by subjecting the mixture to chromatography (in particular, silica gel column chromatography) separation, a mixture may be easily separated and refined.

### (c) A carbon nanoring having 14 or more p-phenylene groups

By using Compound (3c) represented by General Formula (3c): 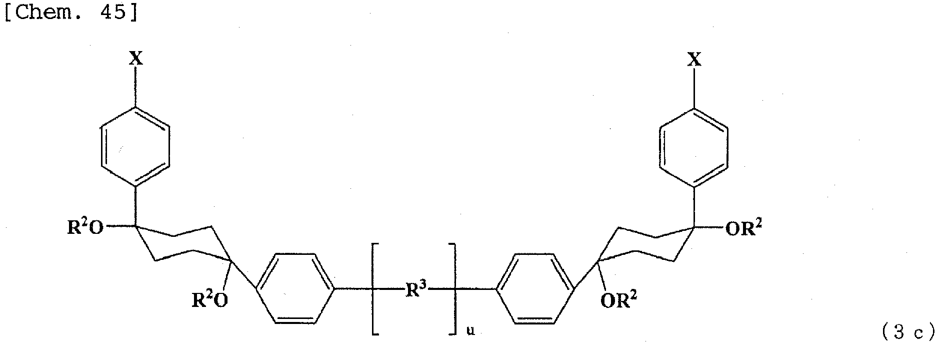 wherein X, R², R³ and u are as defined above,
as Compound (3), i.e., as a starting material for a carbon nanoring, a dimer is obtained.

Thereby, Cyclic Compound (1c) represented by General Formula (1c): 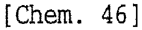 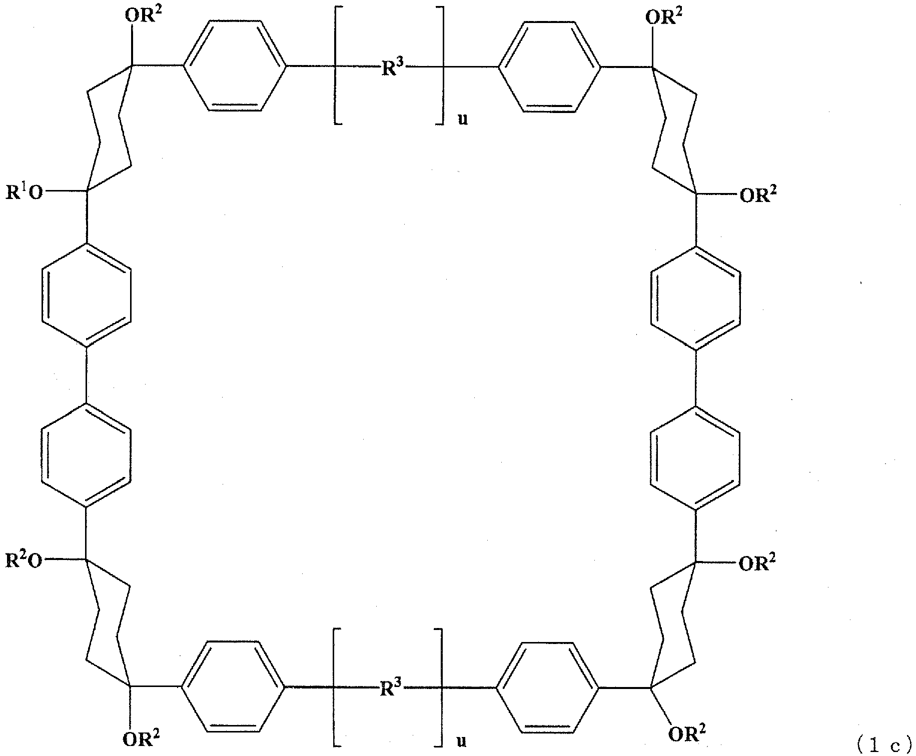 wherein R², R³ and u are as defined above,
is obtained.

Cyclic Compound (1c) is an example of Cyclic Compound (1) in which R¹ is a bivalent group represented by General Formula (5): 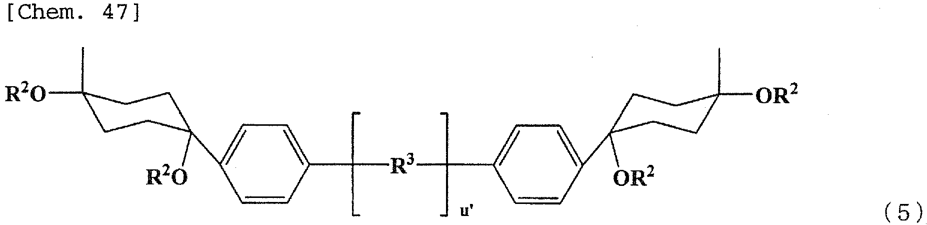 wherein R², R³ and u' are as defined above;
n is 1; and m is 2.

Further, by subjecting Cyclic Compound (1c) to Step (II), a cycloparaphenylene compound that has 14 or more (e.g. 14, 16, etc.) p-phenylene groups and is represented by the following Formula (4c): 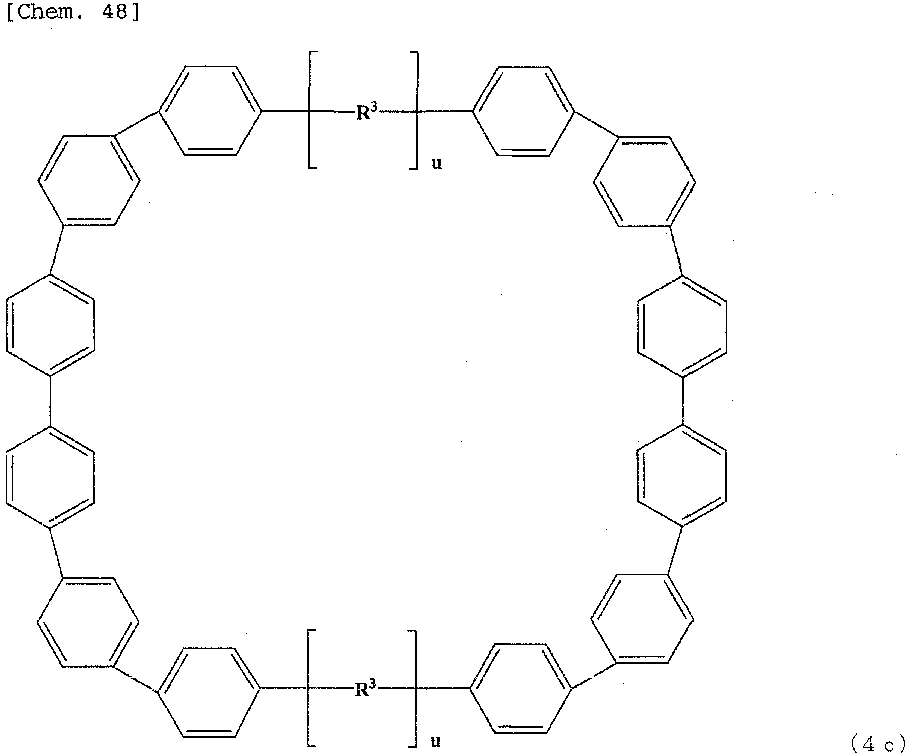 wherein R³ and u are as defined above,
is obtained.

More specifically, the carbon nanoring made of cycloparaphenylenes can be easily produced using the compound represented by General Formula (3c).

### Examples

Hereinafter, the present invention is described in further detail with reference to Examples. However, the scope of the invention is not limited to these Examples. The NMR measurements in the Synthesis Examples and Examples were performed using a nuclear magnetic resonance spectrometer (model name: A-400) produced by JEOL Ltd.

In the Examples, carbon nanorings made of cycloparaphenylenes were produced. First, cis-1,4-bis(4-iodophenyl)-1,4-cyclohexanediol was produced (Synthesis Examples 1 to 3). Thereafter, using this cis-1,4-bis(4-iodophenyl)-1,4-cyclohexanediol, cis-1,4-bis(4-iodophenyl)-1,4-bis(methoxymethylether)cyclohexane was produced (Synthesis Example 4). Subsequently, using this cis-1,4-bis(4-iodophenyl)-1,4-bis(methoxymethylether)cyclohexane, a carbon nanoring was produced (Examples 1 to 3). Each of the cycloparaphenylenes obtained in Examples 1 to 3 was crystallized for structure analysis. In Examples 4 to 5, a cycloparaphenylene compound containing nine p-phenylene groups, and a cycloparaphenylene compound containing sixteen p-phenylene groups were produced, and the analysis thereof was conducted.

### Synthesis Example 1: Synthesis of cis-1,4-bis(4-iodophenyl)-1,4-cyclohexanediol (Compound (3a-1a)) (Part 1)

To a reactor containing an argon gas atmosphere were added 1,4-diiodobenzene (49.5 g, 150 mmol) and anhydrous tetrahydrofuran (300 cm³) at room temperature (25°C), and the resulting solution was cooled to -78°C. Thereafter, a solution of n-butyllithium in hexane (93.8 cm³, 1.6 M, 150 mmol) was slowly added thereto (addition rate: 3 cm³/min). After completion of the addition, the mixture was stirred for 1 hour while the temperature was maintained (-78°C). Subsequently, while the reaction liquid was stirred, a solution of cyclohexane-1,4-diene (5.68 g, 50 mmol) in anhydrous tetrahydrofuran (160 cm³), separately prepared under argon gas atmosphere, was added thereto, and the mixture was reacted at -78°C for 1 hour, and then at room temperature (25°C) for 2 hours. After completion of the reaction, distilled water (100 cm³) and ethyl acetate (500 cm³) were added to the reaction liquid whose temperature had been adjusted to 25°C, and the mixture was placed in a separating funnel. By performing an extraction operation using this separating funnel, the mixture was separated into two layers, i.e., an ethyl acetate layer (i) and an aqueous layer. After the ethyl acetate layer (i) was collected, ethyl acetate (500 cm³) was added to the separated aqueous layer to perform extractive separation again, and an organic layer (ii), which was an ethyl acetate layer, was collected. Ethyl acetate (500 cm³) was added again to the separated aqueous layer to perform extractive separation, and an organic layer (iii), which was an ethyl acetate layer, was collected. Thereafter, a mixed liquid of the ethyl acetate layer (i) and the product-containing organic layers (ii) and (iii) obtained through the additional extraction operations was dried over anhydrous sodium sulfate, and the solvent was distilled off under reduced pressure to yield a crude product. Subsequently, the crude product obtained by the above procedure was subjected to silica gel column chromatography (n-hexane:ethyl acetate = 3:1 → 1:1) to yield a colorless solid (12.6 g). This substance was identified by nuclear magnetic resonance (¹H-NMR) analysis as Compound (3a-1a), i.e., cis-1,4-bis(4-iodophenyl)-1,4-cyclohexanediol, represented by the following General Formula (3a-1a): 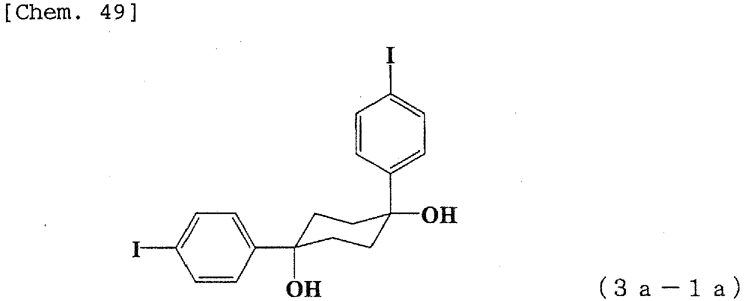 The yield was 48%.

¹H NMR (400 MHz, CDCl₃) δ1.70 (s, 2H), 2.06 (s, 8H), 7.21 (d, J = 8.6 Hz, 4H), 7.68 (d, J = 8.6 Hz, 4H). ¹³C NMR (100 MHz, CDCl₃) δ 35.29, 72.53, 92.27, 127.61, 137.79.

### Synthesis Example 2: Synthesis of cis-1,4-bis(4-iodophenyl)-1,4-cyclohexanediol (Compound (3a-1a)) (Part 2)

To a reactor containing an argon gas atmosphere were added 1,4-diiodobenzene (49.5 g, 150 mmol) and anhydrous tetrahydrofuran (500 cm³) at room temperature (25°C), and the resulting solution was cooled to -78°C. Thereafter, a solution of n-butyllithium in hexane (93.8 cm³, 1.6 M, 150 mmol) was slowly added to the resulting solution (addition rate: 3 cm³/min). After completion of the addition, the mixture was stirred for 1 hour while the temperature was maintained (-78°C). Thereafter, while the reaction liquid was stirred, a solution of cyclohexane-1,4-diene (5.68 g, 50 mmol) in anhydrous tetrahydrofuran (70 cm³), separately prepared under argon gas atmosphere, was added thereto. After completion of the addition, the mixture was stirred and reacted at -78°C for 1 hour, and then at room temperature (25°C) for 2 hours. Thereafter, a saturated ammonium chloride aqueous solution (300 cm³) was added thereto to stop the reaction. Subsequently, the reaction product-containing mixture was placed in a separating funnel. By performing an extraction operation using this separating funnel, the mixture was separated into two layers, i.e., a tetrahydrofuran layer (i) and an aqueous layer. After the tetrahydrofuran layer (i) was collected, ethyl acetate (300 cm³) was added to the separated aqueous layer to perform extractive separation again, and an organic layer, which was a tetrahydrofuran layer, was collected. This operation was repeated 3 times in total to collect organic layers, i.e., tetrahydrofuran layers. Thereafter, a mixed liquid of the tetrahydrofuran layer (i) and the product-containing organic layers obtained through the additional extraction operations (three operations) was washed with a saturated ammonium chloride aqueous solution. The washed organic layer was then dried over anhydrous magnesium sulfate, and the solvent was distilled off under reduced pressure to yield a crude product. Subsequently, the crude product obtained by the above procedure was subjected to silica gel column chromatography (chloroform) to yield a colorless solid (14.3 g). This substance was identified by nuclear magnetic resonance (¹H-NMR) analysis as Compound (3a-1a), i.e., (cis-1,4-bis(4-iodophenyl)-1,4-cyclohexanediol), which was the same as that obtained in Synthesis Example 1. The yield was 55%.

### Synthesis Example 3: Synthesis of cis-1,4-bis(4-iodophenyl)-1,4-cyclohexanediol (Compound (3a-1a)) (Part 3)

To a reactor containing an argon gas atmosphere were added dry cesium trichloride (III) (4.93 g, 20.0 mmol) and lithium chloride (1.70 g, 40.1 mmol) at room temperature (25°C). The reactor was depressurized and the content in the reactor was heated at 150°C for 2 hours. Thereafter, while the reactor was still hot, argon gas was introduced into the reactor. Tetrahydrofuran (100 cm³) was then added thereto and the mixture was stirred at room temperature (25°C) for 12 hours. Thereafter, the temperature was cooled to -78°C, thereby preparing a cesium trichloride mixture.

Subsequently, to a reactor containing an argon gas atmosphere were added 1,4-diiodobenzene (6.60 g, 20.0 mmol) and anhydrous tetrahydrofuran (260 cm³) at room temperature (25°C), and the resulting solution was cooled to -78°C. Thereafter, a solution of n-butyllithium in hexane (12.2 cm³, 1.65 M, 20.1 mmol) was slowly added to the resulting solution. After completion of the addition, the mixture was stirred and reacted for 1.5 hours while the temperature was maintained (-78°C). The reaction solution was then added at -78°C to the above-mentioned cesium trichloride mixture, and stirred at -78°C for 1 hour, and then at 0°C for 30 minutes. Thereafter, while the reaction mixture was stirred, a solution of cyclohexane-1,4-diene (898 mg, 8.01 mmol) in anhydrous tetrahydrofuran (20 cm³), separately prepared under argon gas atmosphere, was added thereto. After completion of the addition, the mixture was stirred and reacted at 0°C for 30 minutes. Thereafter, the reaction solution was adjusted to room temperature (25°C), and a saturated ammonium chloride aqueous solution was added thereto to stop the reaction. Ethyl acetate was added to the reaction product-containing mixture to perform extractive separation as in Synthesis Example 1. The organic layer after extraction was subjected to silica gel column chromatography (n-hexane/ethyl acetate) to yield a white solid (3.13 g). This substance was identified by nuclear magnetic resonance (¹H-NMR) analysis as Compound (3a-1a) (cis-1,4-bis(4-iodophenyl)-1,4-cyclohexanediol). The yield was 75%.

### Synthesis Example 4: Synthesis of cis-1,4-bis(4-iodophenyl)-1,4-bis(methoxymethylether)cyclohexane (Compound (3a-1b))

To a reactor containing an argon gas atmosphere were added cis-1,4-bis(4-iodophenyl)-1,4-cyclohexanediol obtained in Synthesis Example 1, 2, or 3 (Compound (3a-1a); 1.67 g, 3.22 mmol), diisopropylethylamine (4.26 cm³, 24.5 mmol), and anhydrous dichloromethane (15 cm³) at room temperature (25°C), and a solution was prepared. Subsequently, while this solution was stirred, methoxymethyl chloride (1.86 cm³, 24.5 mmol) was slowly added thereto (addition rate: 0.2 cm³/min). After completion of the addition, stirring of the mixture was continued at room temperature (25°C) for 19 hours to allow a reaction to take place. After completion of the reaction, a saturated ammonium chloride aqueous solution (30 cm³) was added to the reaction liquid, and the mixture was placed in a separating funnel. By performing an extraction operation using this separating funnel, the mixture was separated into two layers, i.e., a dichloromethane layer (i) and an aqueous layer. After the dichloromethane layer (i) was collected, dichloromethane (20 cm³) was added to the separated aqueous layer to perform extractive separation again, and an organic layer, which was a dichloromethane layer, was collected. This operation was repeated 3 times in total to collect organic layers, which were dichloromethane layers. Then, a mixed liquid of the dichloromethane layer (i) and the product-containing organic layers obtained through the additional extraction operations (three operations) was dried over anhydrous sodium sulfate, and the solvent was distilled off under reduced pressure to yield a crude product. Subsequently, the crude product obtained by the above procedure was subjected to silica gel column chromatography (n-hexane:ethyl acetate = 10:1) to yield a colorless solid (1.93 g). This substance was identified by nuclear magnetic resonance (¹H-NMR) analysis as cis-1,4-bis(4-iodophenyl)-1,4-bis(methoxymethylether)cyclohexane (Compound (3a-1b)) represented by the following General Formula (3a-1b):

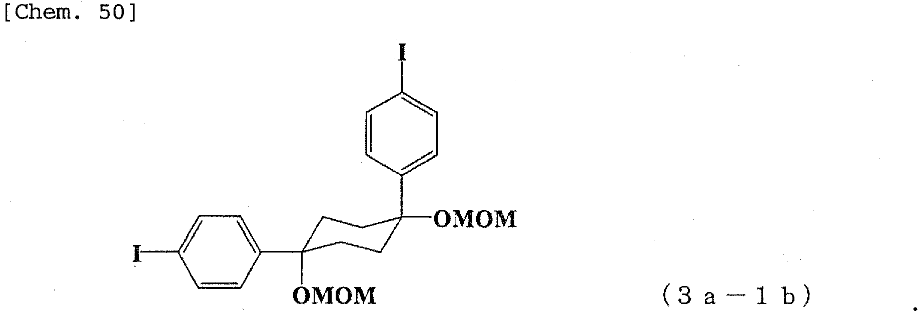

The yield was 98%.

¹H NMR (400 MHz, CDCl₃) δ2.01 (brs, 4H), 2.28 (brs, 4H), 3.39 (s, 6H), 4.41 (s, 4H), 7.16 (d, J = 8.3 Hz, 4H), 7.65 (d, J = 8.7 Hz, 4H). ¹³C NMR (100 MHz, CDCl₃) δ32.74, 56.01, 77.84, 92.15, 93.44, 128.82, 137.49.

### Example 1: Production of [12]cycloparaphenylene (Part 1)

### Production of Cyclic Compound (1a-1a)

To a reactor containing an argon gas atmosphere was added bis(1,5-cyclo-octadiene)nickel(0) (88.0 mg, 0.32 mmol) at room temperature (25°C). Then, cis-1,4-bis(4-iodophenyl)-1,4-bis(methoxymethylether)cyclohexane obtained in Synthesis Example 4 (Compound (3a-1b); 97.3 mg, 0.16 mmol), 2,2'-bipyridine (12.5 mg, 0.08 mmol), 1,5-cyclooctadiene (29.4 mg, 0.27 mmol), and anhydrous 1-methyl-2-pyrrolidinone (3.6 cm³) were added thereto to obtain a solution. Subsequently, the reactor was sealed, and the solution was reacted at 90°C for 24 hours while stirring. Thereafter, the reaction liquid was cooled, ethyl acetate (14.4 cm³) and a saturated aqueous ammonium chloride solution (14.4 cm³) were added thereto, and the mixture was placed in a separating funnel. By performing an extraction operation using this separating funnel, the mixture was separated into two layers, i.e., an ethyl acetate layer (i) and an aqueous layer. After the ethyl acetate layer (i) was collected, ethyl acetate (14.4 cm³) was added to the separated aqueous layer to perform extractive separation again, and an organic layer, which was an ethyl acetate layer, was collected. This operation was repeated 3 times in total, and organic layers, which were ethyl acetate layers, were collected. Then, a mixed liquid of the ethyl acetate layer (i) and the product-containing organic layers obtained through the additional extraction operations (three operations) was washed with a saturated ammonium chloride aqueous solution. Thereafter, the washed organic layer was dried over anhydrous magnesium sulfate, and the solvent was distilled off under reduced pressure to yield a crude product. The crude product obtained by the above procedure was subjected to silica gel preparative thin layer chromatography (n-hexane:ethyl acetate = 1:2). Ethyl acetate was used to extract an organic substance from a spot with an Rf value of 0.25 on the silica gel, and the solvent was distilled off under reduced pressure to yield a colorless solid (11.3 mg). This substance was identified by nuclear magnetic resonance (¹H-NMR) analysis as a tetramer of cis-1,4-bis(4-iodophenyl)-1,4-bis(methoxymethylether) cyclohexane (Cyclic Compound (1a-1a); hereinafter sometimes referred to as "cyclic tetramer") represented by the following General Formula (1a-1a):

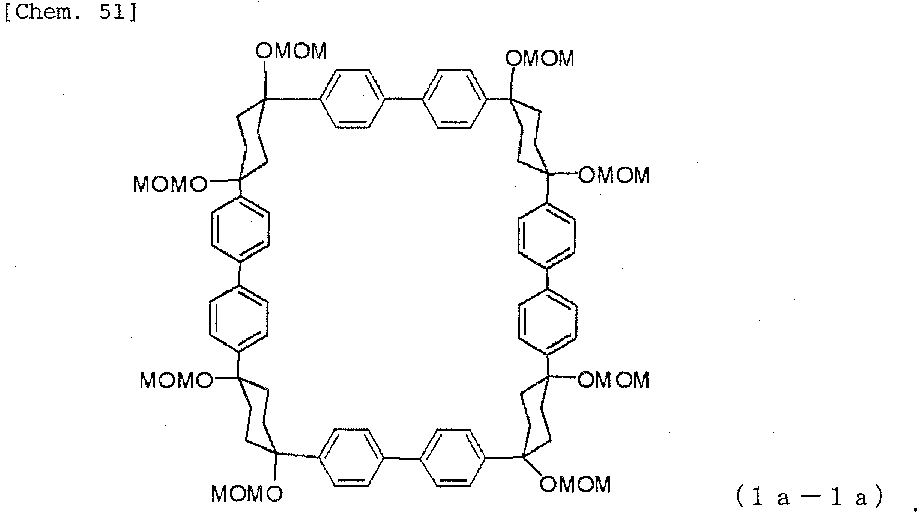

The yield was 20%.

¹H NMR (400 MHz CDCl₃) δ2.16 (brs, 16H), 2.37 (brs, 16H), 3.42 (s, 16H), 4.45 (s, 16H), 7.50 (s, 32H).

When the same procedure was performed without using 1,5-cyclooctadiene, the yield of this cyclic tetramer was 14%.

### Production of [12]cycloparaphenylene

To a dedicated reactor of a microwave synthesizer (trade name: Initiator) produced by Biotage Japan Ltd., were added the cyclic tetramer represented by General Formula (1a-1a) obtained as described above (9.9 mg, 6.98 µmol), para-toluenesulfonic acid monohydrate (1.3 mg, 6.98 µmol), and meta-xylene (1.4 cm³) at room temperature (25°C), and the reactor was sealed to prepare a solution. Thereafter, the maximum microwave radiation emission of the above-mentioned synthesizer was adjusted to 400 W, and the solution was heated at 150°C for 30 minutes while stirring. Subsequently, the reaction liquid was cooled, and dichloromethane (2.0 cm³) and ethyl acetate (2.0 cm³) were added thereto. After insoluble matter was removed by filtration using a suction filter, the solvent was distilled off from the filtrate under reduced pressure to yield a crude product. The crude product obtained by the above procedure was subjected to silica gel preparative thin layer chromatography (n-hexane:chloroform = 1:1). Ethyl acetate was used to extract an organic substance from a spot with an Rf value of 0.37 on the silica gel, and the solvent was distilled off under reduced pressure to yield a light yellow solid (1.2 mg). This substance was identified by nuclear magnetic resonance (¹H-NMR) analysis as [12]cycloparaphenylene (amorphous) containing twelve p-phenylene groups, represented by the following General Formula (4a-1):

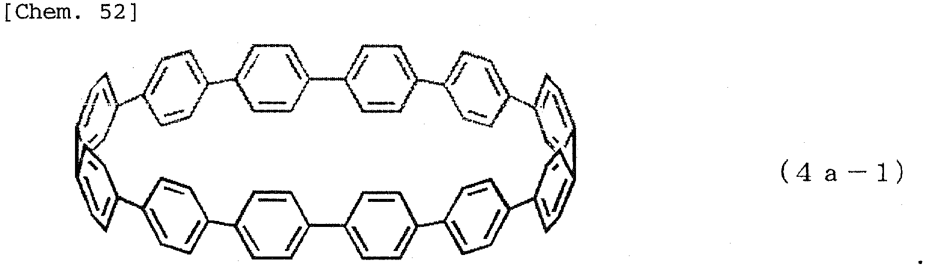

The yield was 19%.

¹H NMR (400 MHz CDCl₃) δ7.61 (s, 48H). ¹³C NMR (100 MHz, CDCl₃) δ 127.33, 138.52.

### Example 2: Production of [12]cycloparaphenylene (Part 2)

In a reactor, the cyclic tetramer represented by General Formula (1a-1a) obtained as described above (9.3 mg, 6.56 µmol) was dissolved in meta-xylene (1.4 cm³), and concentrated sulfuric acid (1.3 mg, 131 µmol) was added at room temperature (25°C) to this solution. Subsequently, the mixture was heated at reflux in an oil bath at 150°C for 24 hours. Thereafter, the reaction liquid was cooled, and dichloromethane (1.9 cm³) and ethyl acetate (1.9 cm³) were added thereto. After insoluble matter was removed by filtration using a suction filter, the solvent was distilled off from the filtrate under reduced pressure to yield a crude product. The crude product obtained by the above procedure was subjected to silica gel preparative thin layer chromatography (n-hexane:chloroform = 1:1). Ethyl acetate was used to extract an organic substance from a spot with an Rf value of 0.37 on the silica gel, and the solvent was distilled off under reduced pressure to yield a light yellow solid (1.2 mg). This substance was identified by nuclear magnetic resonance (¹H-NMR) analysis as [12]cycloparaphenylene (amorphous) containing twelve p-phenylene groups, represented by General Formula (4a-1) above. The yield was 20%.

### Example 3: Production of [12]cycloparaphenylene (Part 3)

To a reactor were added the cyclic tetramer represented by General Formula (1a-1a) obtained as described above (14.2 mg, 10.0 µmol), meta-xylene (1.5 cm³), dimethylsulfoxide (1 cm³), and sodium hydrogen sulfate monohydrate (28.2 mg, 204 µmol) at room temperature (25°C). Subsequently, the mixture was heated at reflux in an oil bath at 150°C for 60 hours. Thereafter, the reaction liquid was cooled, chloroform was added thereto, and the mixture was placed in a separating funnel. By performing an extraction operation using this separating funnel, the chloroform layer was collected, dried over anhydrous sodium sulfate, and the solvent was distilled off under reduced pressure to yield a crude product. The crude product obtained by the above procedure was subjected to silica gel preparative thin layer chromatography (methylene chloride:n-hexane = 1:1). Ethyl acetate was used to extract an organic substance from a spot with an Rf value of 0.37 on the silica gel, and the solvent was distilled off under reduced pressure to yield a white solid (5.9 mg). This substance was identified by nuclear magnetic resonance (¹H-NMR) analysis as [12]cycloparaphenylene (amorphous) containing twelve p-phenylene groups, represented by General Formula (4a-1) above. The yield was 65%.

### Reference Example 1: Crystallization of [12]cycloparaphenylene

[12]Cycloparaphenylene (amorphous) obtained in Example 1, 2, or 3 above, and chloroform, were placed in a reactor to obtain a saturated solution. The reactor was then left open to allow it to stand in n-hexane vapor (25°C, 24 hours), thereby obtaining a crystal of [12]cycloparaphenylene.

### Reference Example 2: X-ray structural analysis of [12]cycloparaphenylene crystal

Using a CCD single crystal automatic X-ray diffractometer (trade name: Saturn) produced by Rigaku Corporation, X-ray structural analysis of the [12]cycloparaphenylene crystal was performed. Table 1 shows the results.

**Table 1**

| | [12]CPP (4a-1) |
|---|---|
| formula | C₈₄H₇₆ |
| fw | 1085.45 |
| T (K) | 123(2) |
| λ (Å) | 0.7107 |
| cryst syst | Monoclinic |
| space group | *P*2₁/c |
| a, (Å) | 19.61(4) |
| b, (Å) | 8.374(15) |
| c, (Å) | 21.14(4) |
| *α*, (deg) | 90 |
| *β*, (deg) | 105.841(20) |
| *γ*, (deg) | 90 |
| *V,* (Å³) | 3339(11) |
| *Z* | 2 |
| D_{calc}, (g / cm³) | 1.080 |
| *µ* (mm⁻¹) | 0.061 |
| F (000) | 1160 |
| cryst size (mm) | 0.15x0.15x0.10 |
| 2 *θ* range, (deg) | 3.15-25.00 |
| reflns collected | 28741 |
| indep refins/Rᵢₙₜ | 5754/0.0821 |
| params | 433 |
| GOF on *F*² | 1.708 |
| R₁, w*R*₂ [I>2 σ (I)] | 0.2309, 0.5229 |
| *R*₁, w*R*₂ (all data) | 0.2915, 0.5559 |

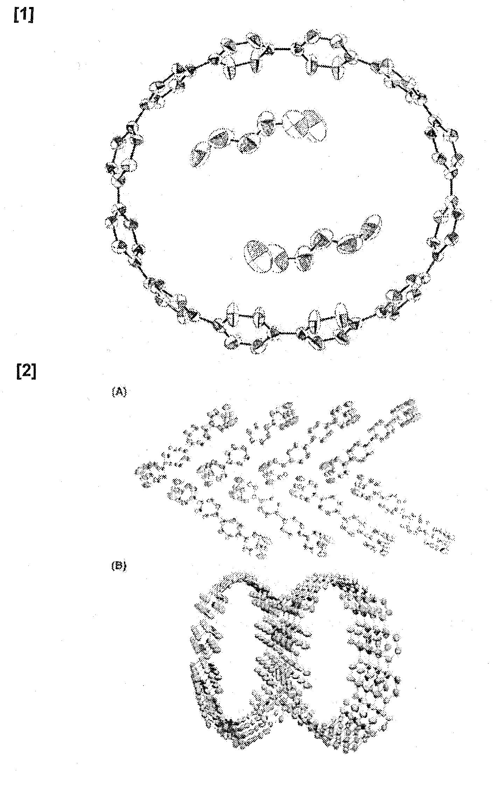

The following findings were obtained from the X-ray structural analysis.
(1) The crystal ring of [12]cycloparaphenylene contains two n-hexane molecules (see Fig. 1).
(2) The crystals of [12]cycloparaphenylene are regularly arranged such that the adjacent crystals are maintained at an angle of 5 to 45 degrees relative to each other (see Fig. 2(A)), and when Fig. 2(A) is seen from the right side, the crystals are in a tube-like shape formed from a large number of rings, due to the arrangement thereof (see Fig. 2(B)).

Figs. 1 and 2 are both produced by the Oak Ridge thermal-ellipsoid plot (ORTEP) program. In Fig. 1, hydrogen atoms are not shown. In Fig. 2, hydrogen atoms and n-hexane molecules are not shown.

### Example 4: Production of [9]cycloparaphenylene

### Production of Cyclic Compound (1b-1)

To a 200-mL round-bottom glass flask containing a stirring bar were added bis(1,5-cyclooctadiene)nickel(0) (452 mg, 1.64 mmol), cis-1,4-bis(4-bromophenyl)-1,4-bis(methoxymethylether)cyclohexane (Compound (3a-1c); 423 mg, 823 µmol) represented by General Formula (3a-1c) obtained as in Synthesis Example 4: 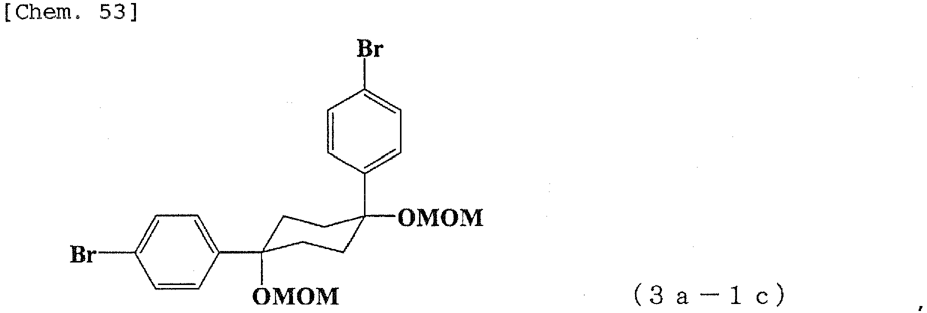 and 2,2'-bipyridine (257 mg, 1.65 mmol). THF (166 mL) was added thereto via syringe. The mixture was then stirred at reflux for 24 hours. After the reaction mixture was cooled to room temperature, this mixture was passed through a silica gel layer and washed with a mixed solvent of EtOAc/CHCl₃. Thereafter, the solvent was removed under reduced pressure. The crude product was subjected to silica gel column chromatography (hexane/EtOAc) to yield a cyclic tetramer (68.1 mg) represented by the following General Formula (1a-1a): 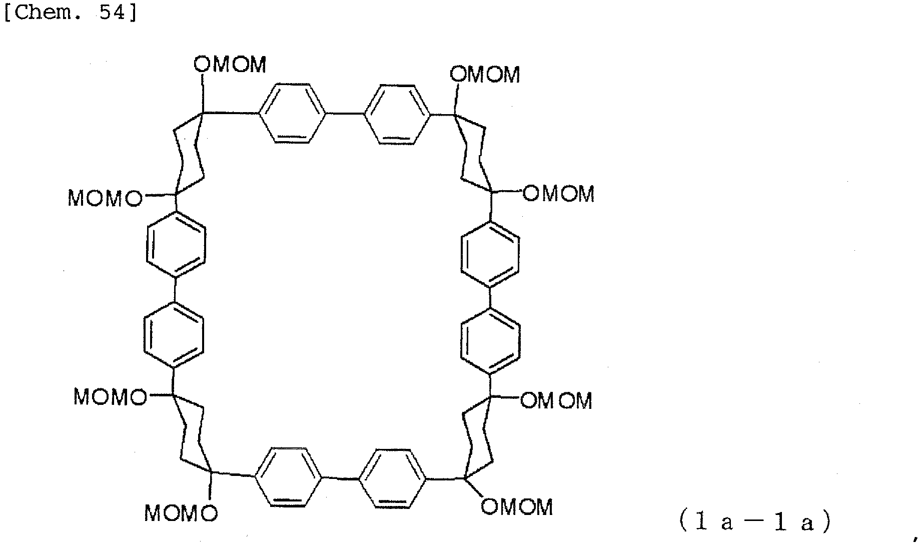 as well as a cyclic trimer (95.5 mg) represented by the following General Formula (1b-1):

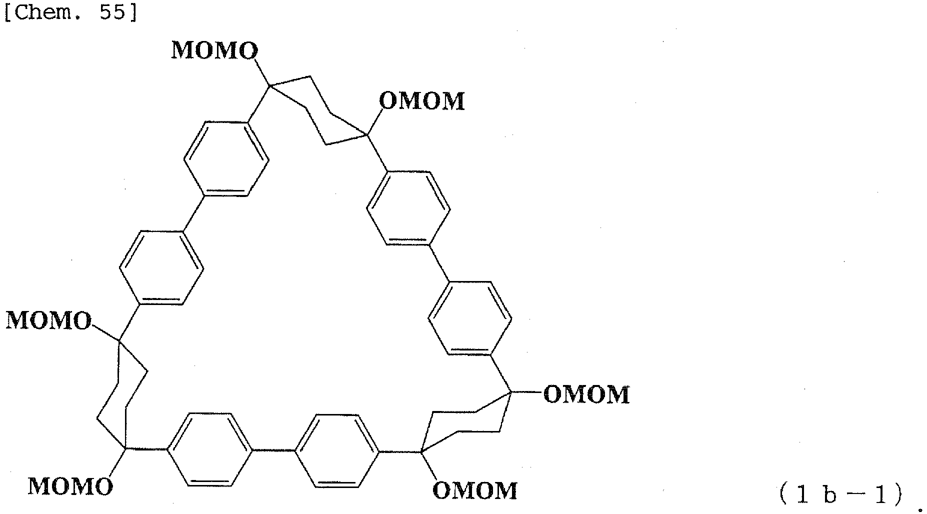

The yield of the cyclic tetramer was 23%, and the yield of the cyclic trimer was 32%. These substances were analyzed by ¹H-NMR and ¹³H-NMR.

### Cyclic trimer:

¹H NMR (600 MHz, 50°C, CDCl₃) δ 2.07 (brs, 12H), 2.28-2.34 (m, 12H), 3.43 (s, 18H), 4.58 (s, 12H), 7.40 (d, J = 8 Hz, 12H), 7.46 (d, J = 8 Hz, 12H);
¹³C NMR (150 MHz, 50° C, CDCl₃) 5 33.3 (CH₂), 55.9 (CH₃), 78.1 (4°), 92.4 (CH₂), 126.8 (CH), 127.3 (CH), 139.4 (4°), 141.2 (4°);
HRMS (FAB) m/z calcd for C₆₆H₇₈NaO₁₂ [M·Na]⁺: 1085.5391, found: 1085.538; mp : 182.3-187.0°C.

### Production of [9]cycloparaphenylene

To a 20-mL Schlenk flask containing a stirring bar and a condenser were added the cyclic trimer (26.6 mg, 25 µmol) represented by General Formula (1b-1) above, sodium hydrogen sulfate monohydrate (69.1 mg, 400 µmol), dry dimethylsulfoxide (1.5 mL), and dry m-xylene (5 mL). The flask was heated at 150°C for 48 hours while stirring. After the reaction mixture was cooled to room temperature, the mixture (reaction liquid) was extracted with CHCl₃, then dried over Na₂SO₄, and the solvent was distilled off under reduced pressure to yield a crude product. Thereafter, TLC (CH₂Cl₂/hexane) was performed to isolate a yellow solid (4.2 mg). This substance was identified by ¹H-NMR and ¹³C-NMR analysis as [9]cycloparaphenylene (amorphous) containing nine p-phenylene groups, represented by General Formula (4b):

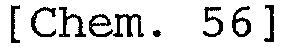

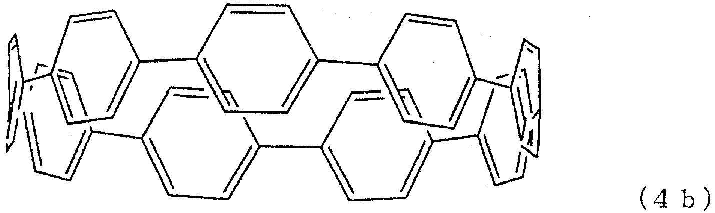

The yield was 24%.

¹H NMR (600 MHz, CDCl₃) δ7.52 (s, 36H);
¹³C NMR (150 MHz, CDCl₃) 5 127.3 (CH), 137.9 (4°); HRMS (MALDI-TOF) m/z calcd for C₅₄H₃₆ [M·]⁺: 684.2817, found: 684.2834.

### Reference Example 3: Crystallization of [9]cycloparaphenylene

[9]cycloparaphenylene (amorphous) obtained in Example 4 above and THF were placed in a reactor to obtain a saturated solution. The reactor was left open to allow it to stand in pentane vapor (10°C, 24 hours), thereby obtaining a crystal of [9]cycloparaphenylene.

### Reference Example 4: X-ray structural analysis of [9]cycloparaphenylene crystal

Using a CCD single crystal automatic X-ray diffractometer (trade name: Saturn) produced by Rigaku Corporation, X-ray structural analysis of the [9]cycloparaphenylene crystal was performed. Table 2 shows the results.

**Table 2**

| | Cyclic Compound (1 b-1) | [9]CPP (4b) |
|---|---|---|
| formula | C₇₀H₈₆O₁₄ | C₆₂H₅₂O₂ |
| fw | 1151.39 | 829.04 |
| T (K) | 103(2) | 103(2) |
| λ (Å) | 0.7107 | 0.7107 |
| cryst syst | Triclinic | Orthorhombic |
| space group | P-1 | *Pnma* |
| a, (A) | 13.421(2) | 20.9873(5) |
| b, (Å) | 14.261(3) | 26.5941(7) |
| c, (Å) | 17.173(3) | 8.0799(2) |
| α, (deg) | 74.195(6) | 90 |
| *β*, (deg) | 76.871(7) | 90 |
| *γ*, (deg) | 85.318(7) | 90 |
| *V,* (Å³) | 3079.2(10) | 4509.7(2) |
| Z | 2 | 4 |
| D_{calc}, (g / cm³) | 1.242 | 1.221 |
| *µ* (mm⁻¹) | 0.085 | 0.072 |
| F (000) | 1236 | 1760 |
| cryst size (mm) | 0.15x0.10x0.05 | 0.20 x 0.10 x 0.03 |
| 2 *θ* range, (deg) | 3.10-25.00 | 2.70-25.00 |
| reflns collected | 20378 | 29023 |
| indep reflns/Rᵢₙₜ | 10540/0.0446 | 4028/0.0716 |
| params | 765 | 340 |
| GOF on *F*² | 1.094 | 1.205 |
| *R*₁, w*R*₂ [I>2 σ (I)] | 0.0683, 0.1428 | 0.0952, 0.2903 |
| *R*₁, w*R*₂ (all data) | 0.1042, 0.1645 | 0.1440, 0.3370 |

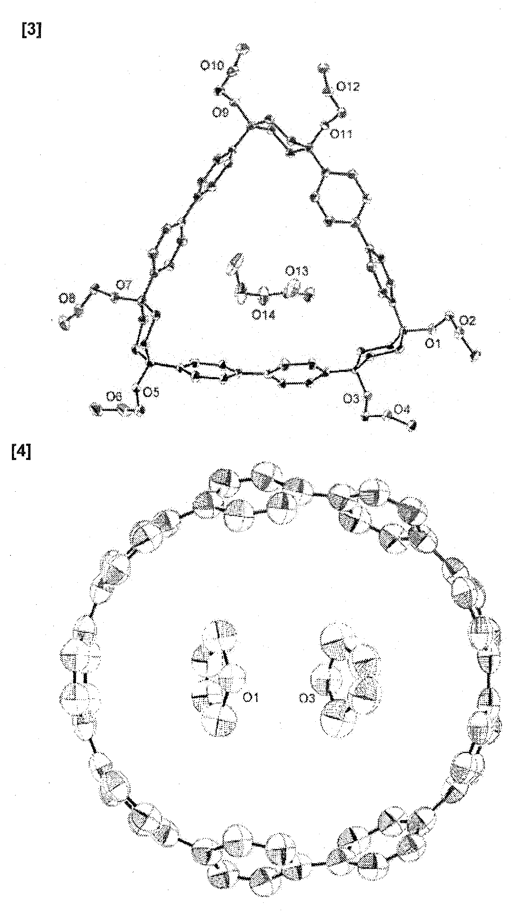

The following findings were obtained from the X-ray structural analysis.
(1) The crystal ring of Cyclic Compound (1b-1) contains ethyl acetate (see Fig. 3).
(2) The crystal ring of [9]cycloparaphenylene contains THF (see Fig. 4).
(3) The crystals of [9]cycloparaphenylene are regularly arranged such that the adjacent crystals are maintained at an angle of 5 to 45 degrees relative to each other, and that the crystal rings are in a tube-like shape formed from a large number of rings due to the arrangement thereof.

Figs. 3 and 4 are both produced by ORTEP. In Fig. 3, hydrogen atoms are not shown. In Fig. 4, all hydrogen atoms and some THF molecules are not shown.

### Synthesis Example 5: Production of Compound (3c-2a)

To a 50-mL round-bottom flask containing a stirring bar were added cesium fluoride (165 mg, 1.1 mmol), cis-1,4-bis(4-bromophenyl)-1,4-bis(methoxymethylether)cyclohexane (Compound (3a-1c)) represented by General Formula (3a-1c) obtained as described in Synthesis Example 4 (521.3 mg, 1 mmol):

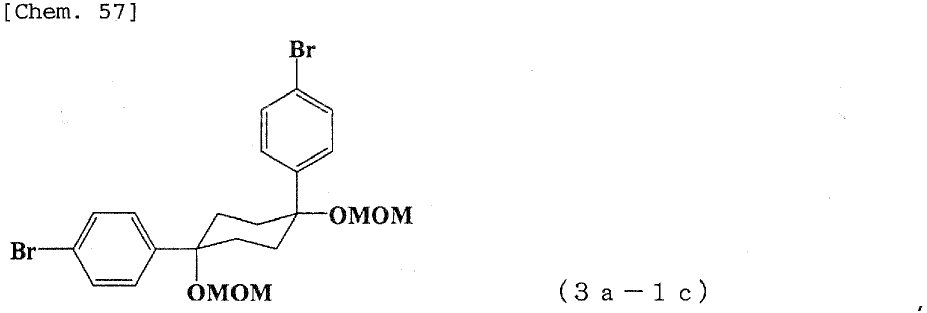

4,4'-biphenyldiboronic acid neopentyl glycol ester (commercially available) represented by General Formula (12) (75.5 mg, 0.2 mmol): 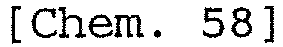 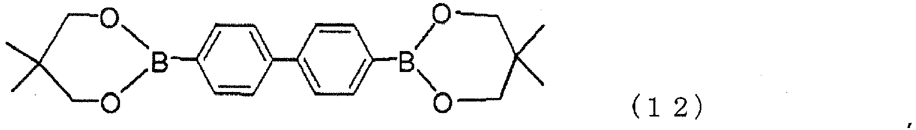 and [Pd(PPh₃)₄] (6.8 mg, 6 µmol), and argon gas was introduced into the flask. Dry THF (60 mL) was added thereto, and the resulting mixture was reacted at 65°C for 26 hours while stirring. Subsequently, the mixture (reaction liquid) in the flask was cooled to room temperature, and was passed through Celite. After the solvent was distilled off from the filtrate under reduced pressure using an evaporator, the residue (concentrate) was purified by silica gel chromatography (hexane/EtOAc) to yield a white solid (126.5 mg). This white solid was identified by nuclear magnetic resonance (¹H-NMR, ¹³C-NMR) analysis and mass spectrometry as Compound (3c-2) represented by the following General Formula (3c-2):

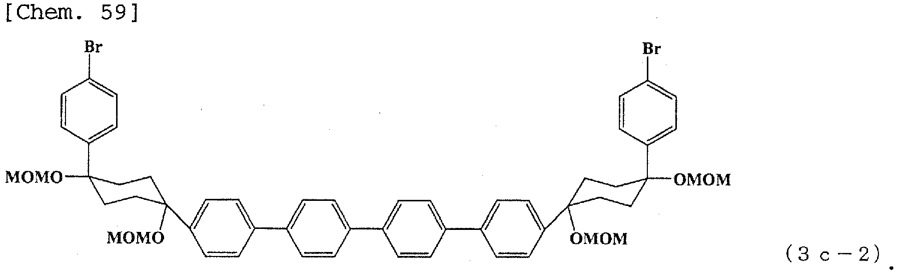

The yield of Compound (3c-2) was 62%.

¹H NMR (400 MHz, CDCl₃) δ2.11 (brs, 8H), 2.34-2.37 (brm, 8H), 3.41 (s, 6H), 3.43 (s, 6H), 4.44 (s, 4H), 4.48 (s, 4H), 7.33 (d, J = 9 Hz, 4H), 7.45 (d, J = 9 Hz, 4H), 7.51 (d, J = 9 Hz, 4H), 7.60 (d, J = 9 Hz, 4H), 7.65 (s, 4H);
¹³C NMR (100 MHz, CDCl₃) δ33.0 (CH₂), 56.0 (CH₃), 77.2 (4°), 77.9 (4°), 78.1 (4°), 92.2 (CH₂), 92.3 (CH₂), 121.7 (4°), 126.9 (CH), 127.4 (CH), 128.7 (4°), 131.5 (CH), 139.5 (4°), 139.8 (4°);
HRMS (FAB) m/z calcd for C₅₆H₆₀Br₂O₈Na [M⁺Na]⁺: 1041.2553, found 1041.2532.

### Example 5: Production of Cyclic Compound (1c-1)

To a 20-mL Schlenk flask containing a stirring bar were added bis(1,5-cyclooctadiene)nickel(0) (55.2 mg, 0.20 mmol), Compound (3c-2) obtained in Synthesis Example 5 (104.6 mg, 0.10 mmol), and 2,2'-bipyridine (31.6 mg, 0.20 mmol). Dry THF (10 mL) was added thereto via syringe. The mixture was then stirred at reflux for 24 hours. After the reaction mixture was cooled to room temperature, this mixture was passed through a silica gel layer, and the solvent was removed under reduced pressure. Thereafter, TLC (EtOAc/CHCl₃) was performed to isolate a white solid (15.1 mg). This substance was identified by ¹H-NMR and ¹³C-NMR analysis as a cyclic compound represented by General Formula (1c-1):

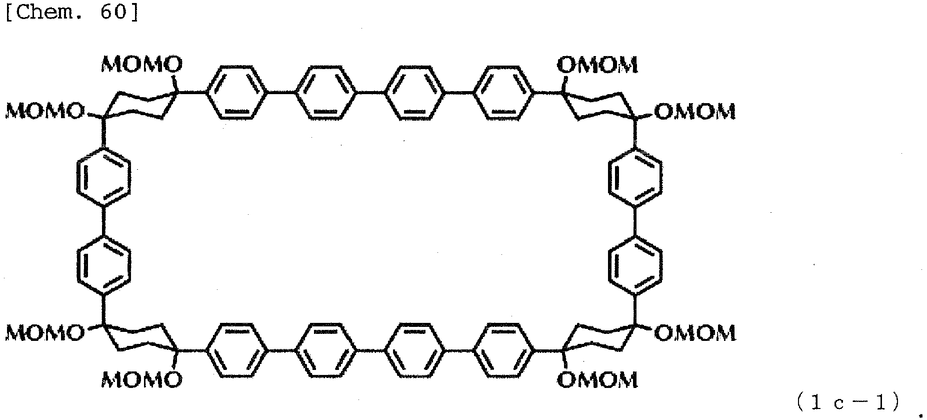

The yield was 17%.

¹H NMR (270 MHz, CDCl3) δ 2.18 (brs, 16H), 2.39 (brs, 16H), 3.43 (s, 12H), 3.45 (s, 12H), 4.47 (s, 8H), 4.50 (s, 8H), 7.50-7.70 (m, 48H);
¹³C NMR (98.5 MHz, CDCl3) 5 33.0 (CH2), 56.0 (CH3), 78.1 (4o), 92.2 (CH2), 126.8 (CH), 127.3 (CH), 139.5 (CH), 139.7 (4o);
HRMS (FAB) m/z calcd for C112H120O16Na [M+Na]+: 1743.8474, found 1743.8496.

When this Cyclic Compound (1c-1) is used, and cyclohexane rings are converted into benzene rings as described in Examples 1 to 4, [16]cycloparaphenylene containing sixteen p-phenylene groups, represented by the following General Formula (4c-1): 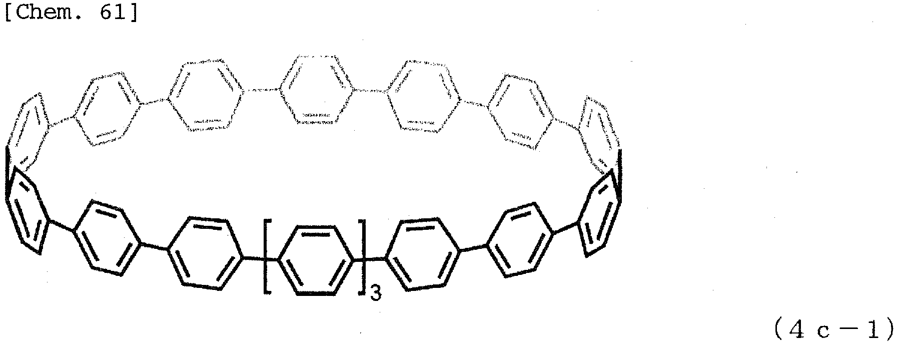 can be obtained.

### Industrial Applicability

The method for producing a carbon nanoring of the present invention is suitable for mass production of carbon nanorings having a desired structure. The carbon nanoring obtained therefrom is suitable as a starting material for a carbon nanotube having a radius within a specific numerical range, and as an electronic material, a luminescent material, and the like.

## Claims

1. A method for producing a cyclic compound represented by General Formula (1): 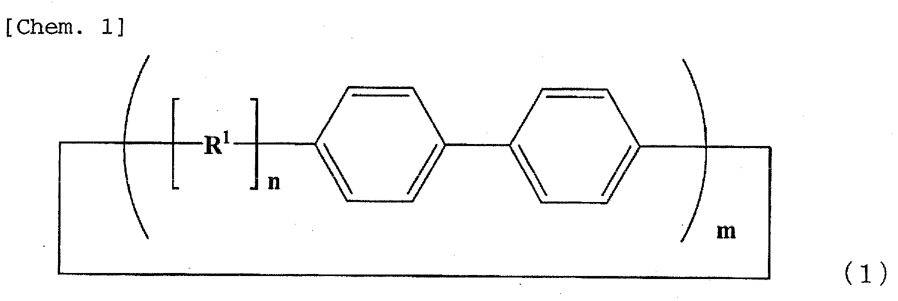 wherein n R¹ is the same or different, and each represents a bivalent aromatic hydrocarbon group, a bivalent alicyclic hydrocarbon group, a bivalent heterocyclic group, or a derivative group thereof; at least one R¹ is a bivalent group represented by Formula (2): 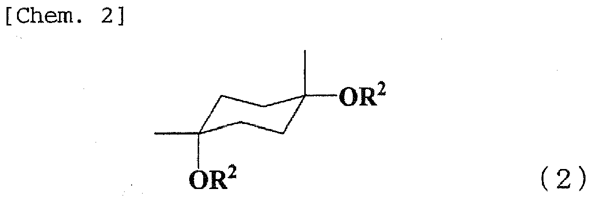 wherein R² is the same or different, and each represents a hydrogen atom or a protecting group for a hydroxy group;
n is an integer of 1 or more; and m is an integer of 2 or more,
the method comprising the step of:
(I) forming a cyclic compound represented by General Formula (1) in the presence of a nickel compound using a compound represented by General Formula (3): 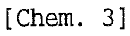 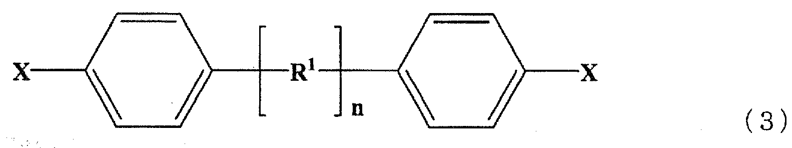 wherein R¹ and n are as defined above; X is the same or different, and each represents a halogen atom.

2. The method for producing the cyclic compound according to claim 1, wherein the compound represented by General Formula (3) is a compound represented by General Formula (3a): 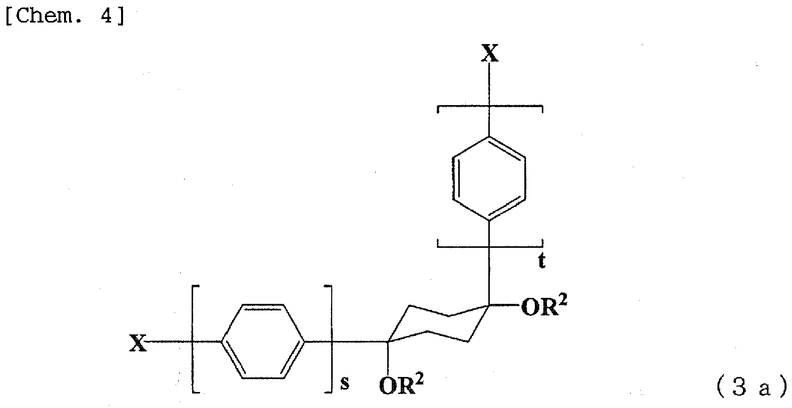 wherein X and R² are as defined above; s is an integer of 1 or more; t is an integer of 1 or more; and s+t=n+1 (n is as defined above).

3. The method for producing the cyclic compound according to claim 1, wherein the compound represented by General Formula (3) is a compound represented by General Formula (3b): 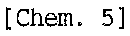 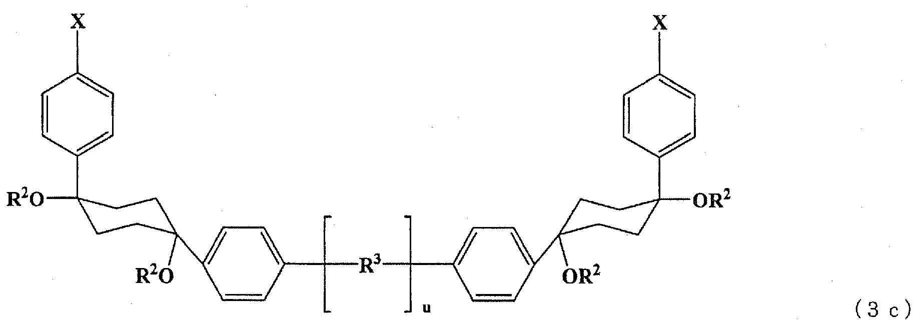 wherein X and R² are as defined above; u R³ is the same or different, and each represents a bivalent aromatic hydrocarbon group, a bivalent alicyclic hydrocarbon group, a bivalent heterocyclic group, or a derivative group thereof; u is an integer of 1 or more; and u = n-4.

4. The method for producing the cyclic compound according to claim 1 or 2,
wherein:
the compound represented by General Formula (3) is Compound (3a-1) represented by General Formula (3a-1): 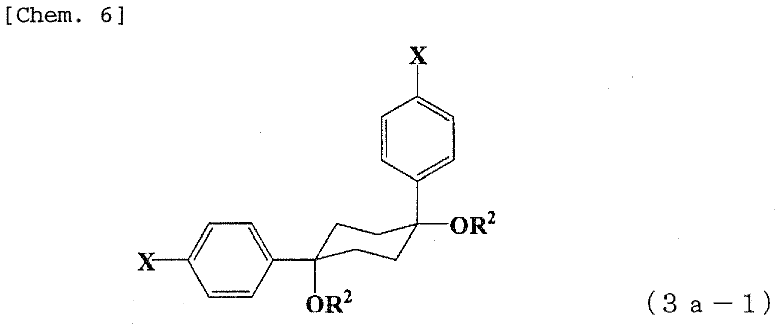 wherein X and R² are as defined above,
and wherein:
m in General Formula (1) is 4.

5. The method for producing the cyclic compound according to claim 1 or 2,
wherein:
the compound represented by General Formula (3) is a compound represented by General Formula (3a-1): 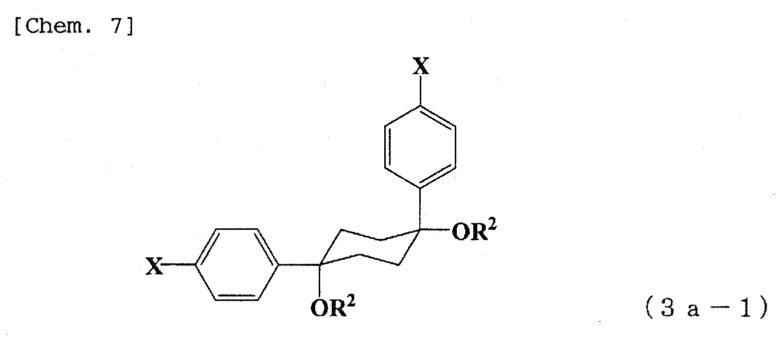 wherein X and R² are as defined above,
and wherein:
m in General Formula (1) is 3.

6. The method for producing the cyclic compound according to claim 3, wherein m in General Formula (1) is 2, and u in General Formula (3b) is 1 or 2.

7. A method for producing a carbon nanoring represented by General Formula (4): 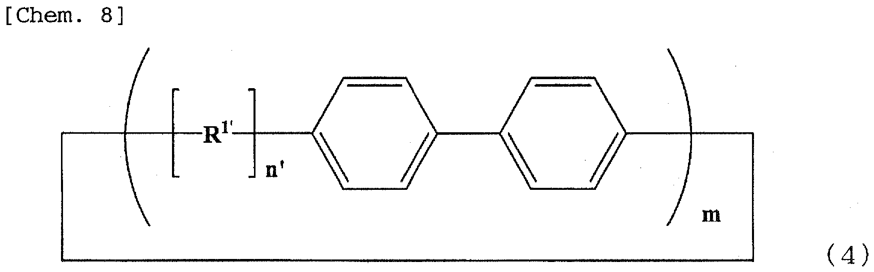 wherein n' R^{1'} is the same or different, and each represents a bivalent aromatic hydrocarbon group, a bivalent alicyclic hydrocarbon group, a bivalent heterocyclic group, or a derivative group thereof; at least one R^{1'} is a p-phenylene group; n' is an integer of 1 or more; and m is an integer of 2 or more,
the method comprising the step of:
(II) converting cyclohexane rings of the cyclic compound obtained by the method according to any one of claims 1 to 6 into benzene rings.

8. The method for producing the carbon nanoring according to claim 7, wherein Step (II) performs an oxidation reaction of Cyclic Compound (1).

9. The method for producing the carbon nanoring according to claim 7 or 8,
wherein:
R^{1'} in General Formula (4) is represented by General Formula (2); n' is 1; and m is 4.

10. The method for producing the carbon nanoring according to claim 7 or 8,
wherein:
R^{1'} in General Formula (4) is represented by General Formula (2); n' is 1; and m is 3.

11. The method for producing the carbon nanoring according to claim 7 or 8,
wherein:
R^{1'} in General Formula (4) is represented by General Formula (5): 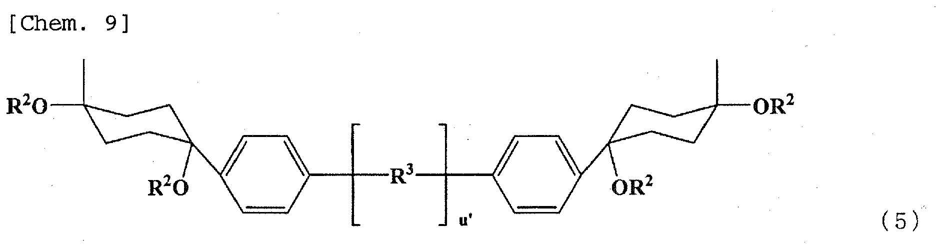 wherein R² and R³ are as defined above; and u' is 1 or 2;
n' is 1 and m is 2.

12. A cyclic compound represented by General Formula (1b): 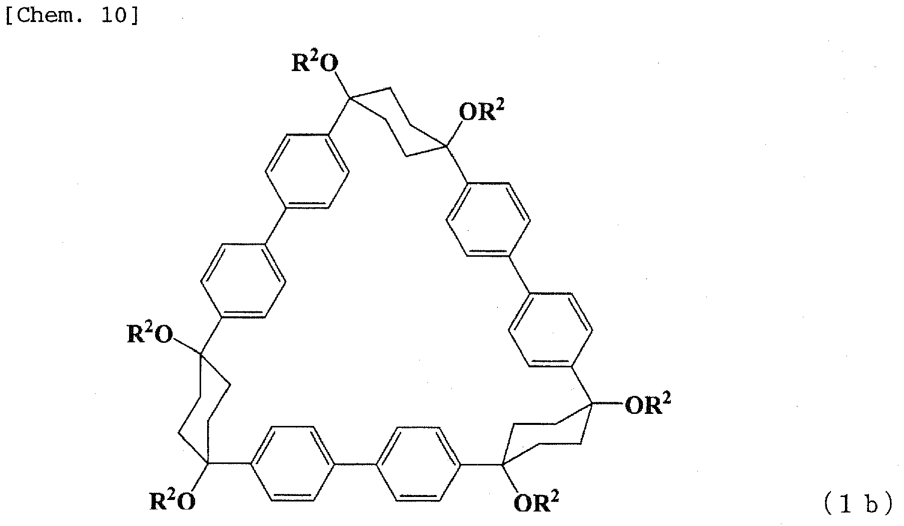 wherein R² is the same or different, and each represents a hydrogen atom or a protecting group for a hydroxy group.

13. A method for producing a carbon nanoring comprising 9 p-phenylene groups, represented by Formula (4b): 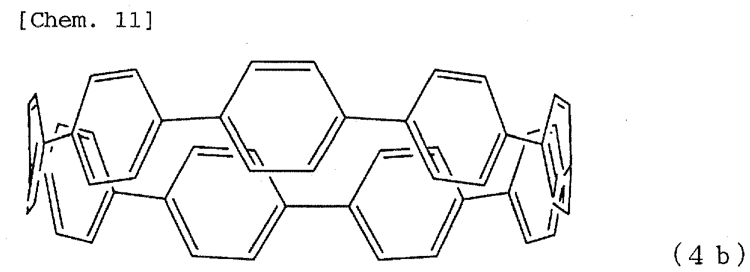 the method comprising the step of:
(IIb) converting cyclohexane rings of a cyclic compound represented by General Formula (1b): 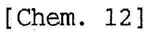 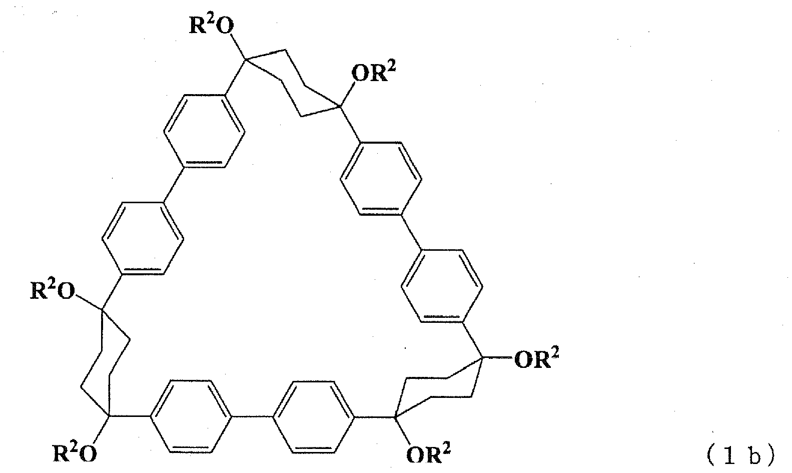 wherein R² is the same or different, and each represents a hydrogen atom or a protecting group for a hydroxy group,
into benzene rings.
